(19) Europäisches Patentamt
European Patent Office
Office européen des brevets

(11) Publication number: **0 393 999 A1**

# (12) EUROPEAN PATENT APPLICATION

(21) Application number: **90304124.2**

(51) Int. Cl.[5]: **C07D 403/12, A01N 47/36**

(22) Date of filing: **18.04.90**

(30) Priority: **20.04.89 AU 3812/89**

(43) Date of publication of application:
**24.10.90 Bulletin 90/43**

(84) Designated Contracting States:
**AT BE CH DE DK ES FR GB GR IT LI LU NL SE**

(71) Applicant: **ICI AUSTRALIA LIMITED**
**1 Nicholson Street**
**Melbourne Victoria 3001(AU)**

(72) Inventor: **Bell, Stephen**
**45 Mirams Street**
**Ascot Vale 3032 Victoria(AU)**
Inventor: **Bonadio, Anna**
**674 Drummond Street**
**North Carlton 3054 Victoria(AU)**
Inventor: **Watson, Keith**
**18 Hamilton Avenue**
**Blackburn 3130 Victoria(AU)**

(74) Representative: **Hardman, Carol Pauline et al**
**Imperial Chemical Industries PLC Legal Department: Patents PO Box 6 Bessemer Road**
**Welwyn Garden City Herts, AL7 1HD(GB)**

## (54) Herbicidal sulfonamide derivatives.

(57) Compounds of the formula

W
‖
$QSO_2NHCNA$
|
R

where Q is an optionally substituted quinazoline ring system, W is oxygen or sulphur, R is one of a range of aliphatic or aromatic hydrocarbon substituents and A is a monocyclic or bicyclic ring system wherein that ring by which A is attached to the remainder of the molecule is a five- or six-membered ring with at least two ring nitrogens. The compounds are effective herbicides.

EP 0 393 999 A1

# HERBICIDAL SULFONAMIDE DERIVATIVES

This invention relates to organic compounds having biological activity and in particular to organic compounds having herbicidal properties and plant growth regulating properties, to processes for the preparation of such compounds, to intermediates useful in the preparation of such compounds and to herbicidal compositions and processes utilizing such compounds.

The use of certain sulfonylurea derivatives as herbicides is known in the art. Thus, for example, the "Pesticide Manual" (C R Worthing Editor, The British Crop Protection Council, 7th Edition 1983) describes the sulfonylurea derivative known commercially as chlorsulfuron [1-(2-chlorophenylsulfonyl)-3-(4-methoxy-6-methyl-1,3,5-triazin-2-yl) urea] and its use as a broadleaf weed herbicide in cereals.

Australian Patent Application No. 23,771/88 describes novel bicyclic heterocyclic sulfonyl ureas and related prior art.

## Summary of the Invention

It has now been found that a new group of bicyclic sulfonylurea derivatives exhibit particularly useful herbicidal activity. They have both pre-emergent and post-emergent application, and some show selective herbicidal activity against broad-leaved and grass weeds in important crops such as maize, rice, wheat, barley, sugarbeet, cotton and soya.

Accordingly the invention provides compounds of the formula I

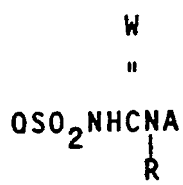

I

wherein Q is one of $Q_1$ to $Q_6$;

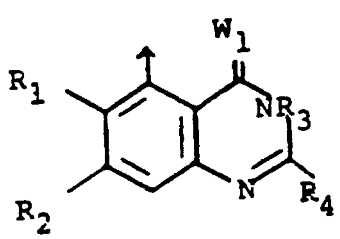

$Q_1$

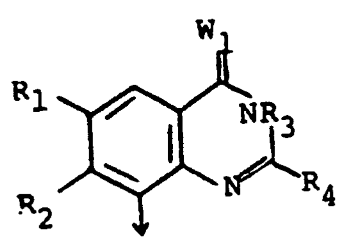

$Q_2$

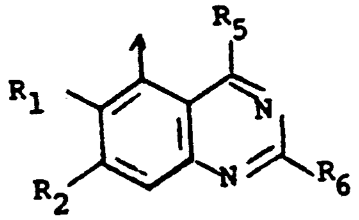

$Q_3$

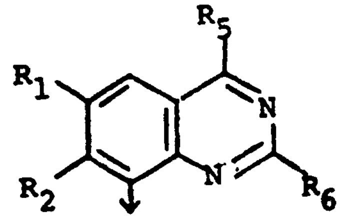

$Q_4$..

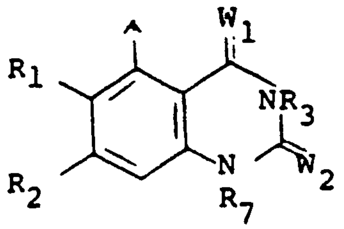

$Q_5$

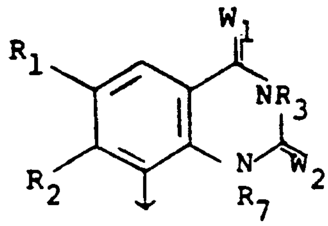

$Q_6$

wherein

W, $W_1$ and $W_2$ are independently O or S;

$R_1$ and $R_2$ are independently hydrogen, halogen, $C_1$-$C_4$ alkyl, $C_1$-$C_4$ haloalkyl, arylthio, aryloxy nitro, $C_1$-$C_4$ alkoxy, $C_1$-$C_4$ haloalkoxy, cyano, carboxy, carbamoyl, N-($C_1$-$C_4$ alkyl)carbamoyl, N,N-di($C_1$-$C_4$ alkyl)-carbamoyl, $C_1$-$C_4$ alkoxycarbonyl, $C_1$-$C_4$ alkylthio, $C_1$-$C_4$ alkylsulfinyl, $C_1$-$C_4$ alkylsulfonyl, sulfamoyl, $C_1$-$C_4$ alkylsulfamoyl, di($C_1$-$C_4$ alkyl) sulfamoyl, amino, $C_1$-$C_4$ alkylamino or di($C_1$-$C_4$ alkyl) amino, $C_1$-$C_4$ acylamino;

R and $R_7$ are each independently selected from hydrogen, optionally substituted $C_1$-$C_4$ alkyl, $C_2$-$C_4$ alkenyl, $C_2$-$C_4$ alkynyl, or optionally substituted aryl or arylalkyl;

$R_3$ is selected from hydrogen, optionally substituted $C_1$-$C_4$ alkyl, $C_2$-$C_4$ alkenyl, $C_2$-$C_4$ alkynyl, optionally substituted aryl or arylalkyl, or $C_2$-$C_5$ alkoxyalkyl;

$R_4$, $R_5$ and $R_6$ are each independently selected from hydrogen, halogen, $C_1$-$C_4$ alkoxy, haloalkoxy, aryloxy, optionally substituted $C_1$-$C_4$ alkyl, $C_2$-$C_4$ alkynyl or optionally substituted aryl or arylalkyl, amino, $C_1$-$C_4$ alkylamino or di($C_1$-$C_4$ alkyl)amino, arylamino, alkylthio, haloalkylthio and arylthio.

A is 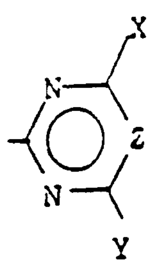 , 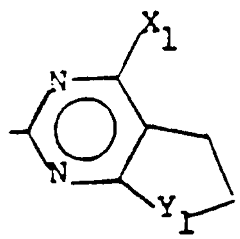 , 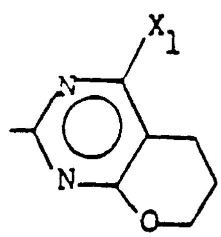 , 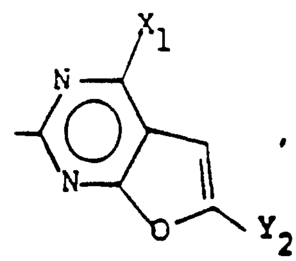 ,

A-1 A-2 A-3 A-4

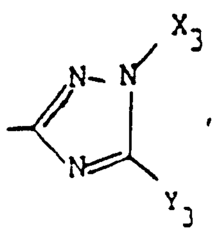 , 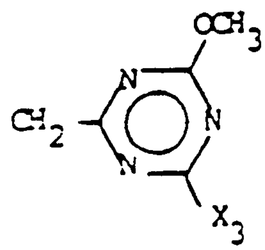 or 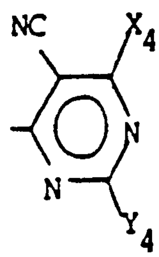

A-5 A-6 A-7

X is $CH_3$, $OCH_3$, $OCH_2CH_3$, Cl, F, Br, I, $OCF_2H$, $CH_2F$ or $CF_3$: Y is H, $CH_3$, $OCH_3$, $OC_2H_5$, $CH_2OCH_3$, $NH_2$, $NHCH_3$, $N(CH_3)_2$, $C_2H_5$, $CF_3$, $SCH_3$, $N(OCH_3)CH_3$, $OCH_2CH=CH_2$, $OCH_2C{\equiv}CH$, $OCH_2CF_3$, cyclopropyl, $OCH_2CH_2OCH_3$, $CH_2SCH_3$, $(C=O)R_8$, $CR_8(OCH_3)_2$,

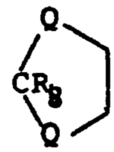 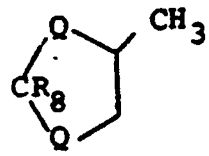 ,

$CR_8(OCH_2CH_3)_2$,

$OCF_2H$, $SCF_2H$, $C \equiv CH$ or $C \equiv CCH_3$

where Q is O or S and $R_8$ is H or $CH_3$; Z is CH, N, $CCH_3$, $CC_2H_5$, CCl or CBr; $Y_1$ is 0 or $CH_2$; $X_3$ is $CH_3$, $OCH_3$, $OC_2H_5$ or $OCF_2H$; $Y_2$ is H or $CH_3$; $X_2$ is $CH_3$, $C_2H_5$ or $CH_2CF_3$; $Y_3$ is $OCH_3$, $OC_2H_5$, $SCH_3$, $SC_2H_5$, $CH_3$ or $CH_2CH_3$; $X_3$ is $CH_3$ or $OCH_3$; $Y_4$ is $CH_3$, $OCH_3$, $OCH_2CH_3$ or Cl; and $X_4$ is $CH_3$, $OCH_3$, $QCH_2CH_3$, $CH_2OCH_3$ or Cl.

The invention also comprises the salts which the compounds of formula I are able to form with amines, alkali metal bases and alkaline earth metal bases, or with quaternary ammonium bases.

In the above definitions, the term "alkyl" used either alone or in compound words such as "alkylthio" or "haloalkyl", denotes straight chain or branched alkyl, e.g., methyl, ethyl, n-propyl, isopropyl or the different butyl isomers.

The term "aryl" used either alone or in the word "arylalkyl" denotes an aromatic ring such as phenyl or pyridyl, optionally substituted by halogen, methoxy, methyl, trifluoromethyl or nitro groups.

Alkoxy denotes methoxy, ethoxy, n-propoxy, isopropoxy and the different butyl isomers.

Alkenyl denotes straight chain or branched alkenes, e.g., vinyl, 1-propenyl, 2-propenyl, 3-propenyl or the different butenyl isomers.

Alkylsulfonyl denotes methylsulfonyl, ethylsulfonyl or the different propylsulfonyl isomers.

Alkylthio, alkylsulfinyl, alkylamino, etc. are defined in an analogous manner. Preferred compounds of the invention include:

1) Those compounds of formula I where

W is O and A is A-1.

2) Compounds of Preferred 1) where

R is hydrogen;

$R_1$ and $R_2$ are independently hydrogen, fluorine, chlorine, bromine, methyl, methoxy, trifluoromethyl, methoxycarbonyl or methylthio; and Y is methyl, methoxy, ethoxy, methoxymethyl, trifluoromethyl, 2,2,2-trifluoroethoxy, dimethoxymethyl or difluoromethoxy.

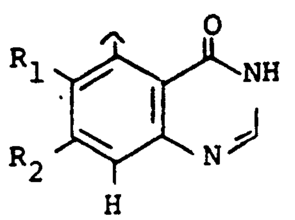

B1

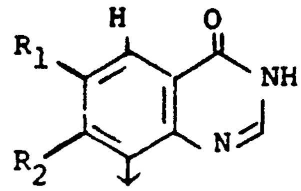

B2

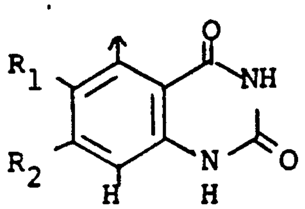

B3

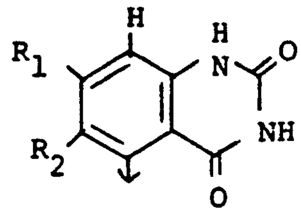

B4

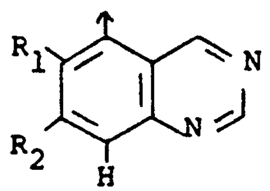

B5

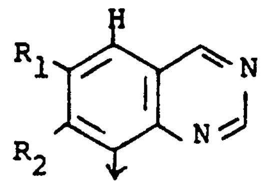

B6

Examples of the basic types of condensed saturated heterocyclic systems which are attached to the sulfonyl end of the sulfonylurea bridge are:
5-sulphonyl-4-oxo-3,4-dihydro-quinazolines $B_1$,
8-sulphonyl-4-oxo-3,4-dihydro-quinazolines B2,
5-sulphonyl-1,2,3,4-tetrahydroquinazolin-2,4-diones B3,
8-sulphonyl-1,2,3,4-tetrahydroquinazolin-2,4-diones B4,
5-sulphonyl-quinazoline B5,
8-sulphonyl-quinazoline B6,

Specific examples of the compounds of the invention include those compounds listed in Table 1.

TABLE 1

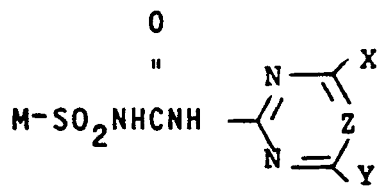

wherein M is:

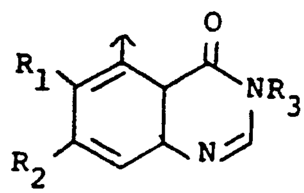

$M_1$

| Compound No. | $R_1$ | $R_2$ | $R_3$ | X | Y | Z |
|---|---|---|---|---|---|---|
| 1 | H | H | H | $CH_3$ | $CH_3$ | CH |
| 2 | H | H | H | $CH_3$ | $OCH_3$ | CH |
| 3 | H | H | H | $OCH_3$ | $OCH_3$ | CH |
| 4 | H | H | H | $CH_3$ | $OCH_3$ | N |
| 5 | Cl | H | H | $CH_3$ | $CH_3$ | CH |
| 6 | Cl | H | H | $CH_3$ | $OCH_3$ | CH |
| 7 | Cl | H | H | $OCH_3$ | $OCH_3$ | CH |
| 8 | Cl | H | H | $CH_3$ | $OCH_3$ | N |
| 9 | $CO_2CH_3$ | H | H | $CH_3$ | $CH_3$ | CH |
| 10 | $CO_2CH_3$ | H | H | $CH_3$ | $OCH_3$ | CH |

TABLE 1 (cont'd)

| Compound No. | $R_1$ | $R_2$ | $R_3$ | X | Y | Z |
|---|---|---|---|---|---|---|
| 11 | $CO_2CH_3$ | H | H | $OCH_3$ | $OCH_3$ | CH |
| 12 | $CO_2CH_3$ | H | H | $CH_3$ | $OCH_3$ | N |
| 13 | $NO_2$ | H | H | $CH_3$ | $CH_3$ | CH |
| 14 | $NO_2$ | H | H | $CH_3$ | $OCH_3$ | CH |
| 15 | $NO_2$ | H | H | $OCH_3$ | $OCH_3$ | CH |
| 16 | $NO_2$ | H | H | $CH_3$ | $OCH_3$ | N |
| 17 | H | H | $CH_3$ | $CH_3$ | $CH_3$ | CH |
| 18 | H | H | $CH_3$ | $CH_3$ | $OCH_3$ | CH |
| 19 | H | H | $CH_3$ | $OCH_3$ | $OCH_3$ | CH |
| 20 | H | H | $CH_3$ | $CH_3$ | $OCH_3$ | N |
| 21 | Cl | H | $CH_3$ | $CH_3$ | $CH_3$ | CH |
| 22 | Cl | H | $CH_3$ | $CH_3$ | $OCH_3$ | CH |
| 23 | Cl | H | $CH_3$ | $OCH_3$ | $OCH_3$ | CH |
| 24 | Cl | H | $CH_3$ | $CH_3$ | $OCH_3$ | N |
| 25 | $CO_2CH_3$ | H | $CH_3$ | $CH_3$ | $CH_3$ | CH |
| 26 | $CO_2CH_3$ | H | $CH_3$ | $CH_3$ | $OCH_3$ | CH |
| 27 | $CO_2CH_3$ | H | $CH_3$ | $OCH_3$ | $OCH_3$ | CH |
| 28 | $CO_2CH_3$ | H | $CH_3$ | $CH_3$ | $OCH_3$ | N |
| 29 | $NO_2$ | H | $CH_3$ | $CH_3$ | $CH_3$ | CH |
| 30 | $NO_2$ | H | $CH_3$ | $CH_3$ | $OCH_3$ | CH |
| 31 | $NO_2$ | H | $CH_3$ | $OCH_3$ | $OCH_3$ | CH |
| 32 | $NO_2$ | H | $CH_3$ | $CH_3$ | $OCH_3$ | N |
| 33 | $OCH_3$ | H | H | $CH_3$ | $CH_3$ | CH |
| 34 | $OCH_3$ | H | H | $CH_3$ | $OCH_3$ | CH |
| 35 | $OCH_3$ | H | H | $OCH_3$ | $OCH_3$ | CH |
| 36 | $OCH_3$ | H | H | $CH_3$ | $OCH_3$ | N |
| 37 | $SCH_3$ | H | H | $CH_3$ | $CH_3$ | CH |
| 38 | $SCH_3$ | H | H | $CH_3$ | $OCH_3$ | CH |
| 39 | $SCH_3$ | H | H | $OCH_3$ | $OCH_3$ | CH |
| 40 | $SCH_3$ | H | H | $CH_3$ | $OCH_3$ | N |

TABLE 1 (cont'd)

| Compound No. | $R_1$ | $R_2$ | $R_3$ | X | Y | Z |
|---|---|---|---|---|---|---|
| 41 | Br | H | H | $CH_3$ | $CH_3$ | CH |
| 42 | Br | H | H | $CH_3$ | $OCH_3$ | CH |
| 43 | Br | H | H | $OCH_3$ | $OCH_3$ | CH |
| 44 | Br | H | H | $CH_3$ | $OCH_3$ | N |
| 45 | $OCH_3$ | H | $CH_3$ | $CH_3$ | $CH_3$ | CH |
| 46 | $OCH_3$ | H | $CH_3$ | $CH_3$ | $OCH_3$ | CH |
| 47 | $OCH_3$ | H | $CH_3$ | $OCH_3$ | $OCH_3$ | CH |
| 48 | $OCH_3$ | H | $CH_3$ | $CH_3$ | $OCH_3$ | N |
| 49 | $SCH_3$ | H | $CH_3$ | $CH_3$ | $CH_3$ | CH |
| 50 | $SCH_3$ | H | $CH_3$ | $CH_3$ | $OCH_3$ | CH |
| 51 | $SCH_3$ | H | $CH_3$ | $OCH_3$ | $OCH_3$ | CH |
| 52 | $SCH_3$ | H | $CH_3$ | $CH_3$ | $OCH_3$ | N |
| 53 | Br | H | $CH_3$ | $CH_3$ | $CH_3$ | CH |
| 54 | Br | H | $CH_3$ | $CH_3$ | $OCH_3$ | CH |
| 55 | Br | H | $CH_3$ | $OCH_3$ | $OCH_3$ | CH |
| 56 | Br | H | $CH_3$ | $CH_3$ | $OCH_3$ | N |
| 57 | H | H | $CH_2OCH_3$ | $CH_3$ | $CH_3$ | CH |
| 58 | H | H | $CH_2OCH_3$ | $CH_3$ | $OCH_3$ | CH |
| 59 | H | H | $CH_2OCH_3$ | $OCH_3$ | $OCH_3$ | CH |
| 60 | H | H | $CH_2OCH_3$ | $CH_3$ | $OCH_3$ | N |
| 61 | Cl | H | $CH_2OCH_3$ | $CH_3$ | $CH_3$ | CH |
| 62 | Cl | H | $CH_2OCH_3$ | $CH_3$ | $OCH_3$ | CH |
| 63 | Cl | H | $CH_2OCH_3$ | $OCH_3$ | $OCH_3$ | CH |
| 64 | Cl | H | $CH_2OCH_3$ | $CH_3$ | $OCH_3$ | N |
| 65 | $CO_2CH_3$ | H | $CH_2OCH_3$ | $CH_3$ | $CH_3$ | CH |
| 66 | $CO_2CH_3$ | H | $CH_2OCH_3$ | $CH_3$ | $OCH_3$ | CH |

TABLE 1 (Cont'd)

| Compound No. | $R_1$ | $R_2$ | $R_3$ | X | Y | Z |
|---|---|---|---|---|---|---|
| 67 | $CO_2CH_3$ | H | $CH_2CH_3$ | $OCH_3$ | $OCH_3$ | CH |
| 68 | $CO_2CH_3$ | H | $CH_2CH_3$ | $CH_3$ | $OCH_3$ | N |
| 69 | $NO_2$ | H | $CH_2CH_3$ | $CH_3$ | $CH_3$ | CH |
| 70 | $NO_2$ | H | $CH_2CH_3$ | $CH_3$ | $OCH_3$ | CH |
| 71 | $NO_2$ | H | $CH_2CH_3$ | $OCH_3$ | $OCH_3$ | CH |
| 72 | $NO_2$ | H | $CH_2CH_3$ | $CH_3$ | $OCH_3$ | N |
| 73 | $OCH_3$ | H | $CH_2CH_3$ | $CH_3$ | $CH_3$ | CH |
| 74 | $OCH_3$ | H | $CH_2CH_3$ | $CH_3$ | $OCH_3$ | CH |
| 75 | $OCH_3$ | H | $CH_2CH_3$ | $OCH_3$ | $OCH_3$ | CH |
| 76 | $OCH_3$ | H | $CH_2CH_3$ | $CH_3$ | $OCH_3$ | N |
| 77 | $SCH_3$ | H | $CH_2CH_3$ | $CH_3$ | $CH_3$ | CH |
| 78 | $SCH_3$ | H | $CH_2CH_3$ | $CH_3$ | $OCH_3$ | CH |
| 79 | $SCH_3$ | H | $CH_2CH_3$ | $OCH_3$ | $OCH_3$ | CH |
| 80 | $SCH_3$ | H | $CH_2CH_3$ | $CH_3$ | $OCH_3$ | N |
| 81 | Br | H | $CH_2CH_3$ | $CH_3$ | $CH_3$ | CH |
| 82 | Br | H | $CH_2CH_3$ | $CH_3$ | $OCH_3$ | CH |
| 83 | Br | H | $CH_2CH_3$ | $OCH_3$ | $OCH_3$ | CH |
| 84 | Br | H | $CH_2CH_3$ | $CH_3$ | $OCH_3$ | N |
| 85 | $NC_2H_5$ | H | $CH_3$ | $OCH_3$ | $OCH_3$ | CH |
| 86 | $SC_6H_5$ | H | $CH_3$ | $OCH_3$ | $OCH_3$ | CH |

TABLE 2

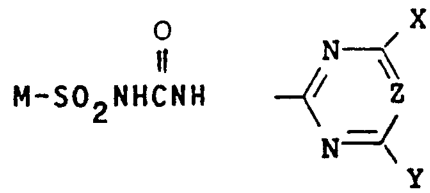

wherein M is:

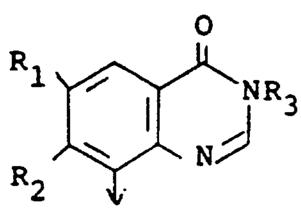

$M_2$

| Compound No. | $R_1$ | $R_2$ | $R_3$ | X | Y | Z |
|---|---|---|---|---|---|---|
| 1 | H | H | H | $CH_3$ | $CH_3$ | CH |
| 2 | H | H | H | $CH_3$ | $OCH_3$ | CH |
| 3 | H | H | H | $OCH_3$ | $OCH_3$ | CH |
| 4 | H | H | H | $CH_3$ | $OCH_3$ | N |
| 5 | H | Cl | H | $CH_3$ | $CH_3$ | CH |
| 6 | H | Cl | H | $CH_3$ | $OCH_3$ | CH |
| 7 | H | Cl | H | $OCH_3$ | $OCH_3$ | CH |
| 8 | H | Cl | H | $CH_3$ | $OCH_3$ | N |
| 9 | H | $CO_2CH_3$ | H | $CH_3$ | $CH_3$ | CH |
| 10 | H | $CO_2CH_3$ | H | $CH_3$ | $OCH_3$ | CH |

TABLE 2 (cont'd)

| Compound No. | $R_1$ | $R_2$ | $R_3$ | X | Y | Z |
|---|---|---|---|---|---|---|
| 11 | H | $CO_2CH_3$ | H | $OCH_3$ | $OCH_3$ | CH |
| 12 | H | $CO_2CH_3$ | H | $CH_3$ | $OCH_3$ | N |
| 13 | H | $NO_2$ | H | $CH_3$ | $CH_3$ | CH |
| 14 | H | $NO_2$ | H | $CH_3$ | $OCH_3$ | CH |
| 15 | H | $NO_2$ | H | $OCH_3$ | $OCH_3$ | CH |
| 16 | H | $NO_2$ | H | $CH_3$ | $OCH_3$ | N |
| 17 | H | H | $CH_3$ | $CH_3$ | $CH_3$ | CH |
| 18 | H | H | $CH_3$ | $CH_3$ | $OCH_3$ | CH |
| 19 | H | H | $CH_3$ | $OCH_3$ | $OCH_3$ | CH |
| 20 | H | H | $CH_3$ | $CH_3$ | $OCH_3$ | N |
| 21 | H | Cl | $CH_3$ | $CH_3$ | $CH_3$ | CH |
| 22 | H | Cl | $CH_3$ | $CH_3$ | $OCH_3$ | CH |
| 23 | H | Cl | $CH_3$ | $OCH_3$ | $OCH_3$ | CH |
| 24 | H | Cl | $CH_3$ | $CH_3$ | $OCH_3$ | N |
| 25 | H | $CO_2CH_3$ | $CH_3$ | $CH_3$ | $CH_3$ | CH |
| 26 | H | $CO_2CH_3$ | $CH_3$ | $CH_3$ | $OCH_3$ | CH |
| 27 | H | $CO_2CH_3$ | $CH_3$ | $OCH_3$ | $OCH_3$ | CH |
| 28 | H | $CO_2CH_3$ | $CH_3$ | $CH_3$ | $OCH_3$ | N |
| 29 | H | $NO_2$ | $CH_3$ | $CH_3$ | $CH_3$ | CH |
| 30 | H | $NO_2$ | $CH_3$ | $CH_3$ | $OCH_3$ | CH |
| 31 | H | $NO_2$ | $CH_3$ | $OCH_3$ | $OCH_3$ | CH |
| 32 | H | $NO_2$ | $CH_3$ | $CH_3$ | $OCH_3$ | N |
| 33 | H | $OCH_3$ | H | $CH_3$ | $CH_3$ | CH |
| 34 | H | $OCH_3$ | H | $CH_3$ | $OCH_3$ | CH |
| 35 | H | $OCH_3$ | H | $OCH_3$ | $OCH_3$ | CH |
| 36 | H | $OCH_3$ | H | $CH_3$ | $OCH_3$ | N |
| 37 | H | $SCH_3$ | H | $CH_3$ | $CH_3$ | CH |
| 38 | H | $SCH_3$ | H | $CH_3$ | $OCH_3$ | CH |
| 39 | H | $SCH_3$ | H | $OCH_3$ | $OCH_3$ | CH |
| 40 | H | $SCH_3$ | H | $CH_3$ | $OCH_3$ | N |

TABLE 2 (cont'd)

| Compound No. | $R_1$ | $R_2$ | $R_3$ | X | Y | Z |
|---|---|---|---|---|---|---|
| 41 | H | Br | H | $CH_3$ | $CH_3$ | CH |
| 42 | H | Br | H | $CH_3$ | $OCH_3$ | CH |
| 43 | H | Br | H | $OCH_3$ | $OCH_3$ | CH |
| 44 | H | Br | H | $CH_3$ | $OCH_3$ | N |
| 45 | H | $OCH_3$ | $CH_3$ | $CH_3$ | $CH_3$ | CH |
| 46 | H | $OCH_3$ | $CH_3$ | $CH_3$ | $OCH_3$ | CH |
| 47 | H | $OCH_3$ | $CH_3$ | $OCH_3$ | $OCH_3$ | CH |
| 48 | H | $OCH_3$ | $CH_3$ | $CH_3$ | $OCH_3$ | N |
| 49 | H | $SCH_3$ | $CH_3$ | $CH_3$ | $CH_3$ | CH |
| 50 | H | $SCH_3$ | $CH_3$ | $CH_3$ | $OCH_3$ | CH |
| 51 | H | $SCH_3$ | $CH_3$ | $OCH_3$ | $OCH_3$ | CH |
| 52 | H | $SCH_3$ | $CH_3$ | $CH_3$ | $OCH_3$ | N |
| 53 | H | Br | $CH_3$ | $CH_3$ | $CH_3$ | CH |
| 54 | H | Br | $CH_3$ | $CH_3$ | $OCH_3$ | CH |
| 55 | H | Br | $CH_3$ | $OCH_3$ | $OCH_3$ | CH |
| 56 | H | Br | $CH_3$ | $CH_3$ | $OCH_3$ | N |
| 57 | H | H | $CH_2OCH_3$ | $CH_3$ | $CH_3$ | CH |
| 58 | H | H | $CH_2OCH_3$ | $CH_3$ | $OCH_3$ | CH |
| 59 | H | H | $CH_2OCH_3$ | $OCH_3$ | $OCH_3$ | CH |
| 60 | H | H | $CH_2OCH_3$ | $CH_3$ | $OCH_3$ | N |
| 61 | H | Cl | $CH_2OCH_3$ | $CH_3$ | $CH_3$ | CH |
| 62 | H | Cl | $CH_2OCH_3$ | $CH_3$ | $OCH_3$ | CH |
| 63 | H | Cl | $CH_2OCH_3$ | $OCH_3$ | $OCH_3$ | CH |
| 64 | H | Cl | $CH_2OCH_3$ | $CH_3$ | $OCH_3$ | N |
| 65 | H | $CO_2CH_3$ | $CH_2OCH_3$ | $CH_3$ | $CH_3$ | CH |
| 66 | H | $CO_2CH_3$ | $CH_2OCH_3$ | $CH_3$ | $OCH_3$ | CH |

TABLE 2 (cont'd)

| Compound No. | $R_1$ | $R_2$ | $R_3$ | X | Y | Z |
|---|---|---|---|---|---|---|
| 67 | H | $CO_2CH_3$ | $CH_2OCH_3$ | $OCH_3$ | $OCH_3$ | CH |
| 68 | H | $CO_2CH_3$ | $CH_2OCH_3$ | $CH_3$ | $OCH_3$ | N |
| 69 | H | $NO_2$ | $CH_2OCH_3$ | $CH_3$ | $CH_3$ | CH |
| 70 | H | $NO_2$ | $CH_2OCH_3$ | $CH_3$ | $OCH_3$ | CH |
| 71 | H | $NO_2$ | $CH_2OCH_3$ | $OCH_3$ | $OCH_3$ | CH |
| 72 | H | $NO_2$ | $CH_2OCH_3$ | $CH_3$ | $OCH_3$ | N |
| 73 | H | $OCH_3$ | $CH_2OCH_3$ | $CH_3$ | $CH_3$ | CH |
| 74 | H | $OCH_3$ | $CH_2OCH_3$ | $CH_3$ | $OCH_3$ | CH |
| 75 | H | $OCH_3$ | $CH_2OCH_3$ | $OCH_3$ | $OCH_3$ | CH |
| 76 | H | $OCH_3$ | $CH_2OCH_3$ | $CH_3$ | $OCH_3$ | N |
| 77 | H | $SCH_3$ | $CH_2OCH_3$ | $CH_3$ | $CH_3$ | CH |
| 78 | H | $SCH_3$ | $CH_2OCH_3$ | $CH_3$ | $OCH_3$ | CH |
| 79 | H | $SCH_3$ | $CH_2OCH_3$ | $OCH_3$ | $OCH_3$ | CH |
| 80 | H | $SCH_3$ | $CH_2OCH_3$ | $CH_3$ | $OCH_3$ | N |
| 81 | H | Br | $CH_2OCH_3$ | $CH_3$ | $CH_3$ | CH |
| 82 | H | Br | $CH_2OCH_3$ | $CH_3$ | $OCH_3$ | CH |
| 83 | H | Br | $CH_2OCH_3$ | $OCH_3$ | $OCH_3$ | CH |
| 84 | H | Br | $CH_2OCH_3$ | $CH_3$ | $OCH_3$ | N |

TABLE 3

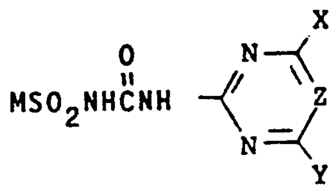

wherein M is :

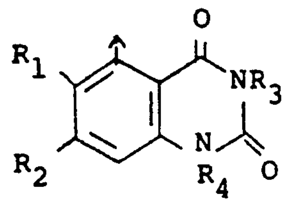

$M_3$

| Compound No. | $R_1$ | $R_2$ | $R_3$ | $R_4$ | X | Y | Z |
|---|---|---|---|---|---|---|---|
| 1 | H | H | H | H | $CH_3$ | $CH_3$ | CH |
| 2 | H | H | H | H | $CH_3$ | $OCH_3$ | CH |
| 3 | H | H | H | H | $OCH_3$ | $OCH_3$ | CH |
| 4 | H | H | H | H | $CH_3$ | $OCH_3$ | N |
| 5 | H | Cl | H | H | $CH_3$ | $CH_3$ | CH |
| 6 | H | Cl | H | H | $CH_3$ | $OCH_3$ | CH |
| 7 | H | Cl | H | H | $OCH_3$ | $OCH_3$ | CH |
| 8 | H | Cl | H | H | $CH_3$ | $OCH_3$ | N |
| 9 | Cl | H | H | H | $CH_3$ | $CH_3$ | CH |
| 10 | Cl | H | H | H | $CH_3$ | $OCH_3$ | CH |
| 11 | Cl | H | H | H | $OCH_3$ | $OCH_3$ | CH |
| 12 | Cl | H | H | H | $CH_3$ | $OCH_3$ | N |
| 13 | Br | H | H | H | $CH_3$ | $CH_3$ | CH |
| 14 | Br | H | H | H | $CH_3$ | $OCH_3$ | CH |
| 15 | Br | H | H | H | $OCH_3$ | $OCH_3$ | CH |
| 16 | Br | H | H | H | $CH_3$ | $OCH_3$ | N |
| 17 | $CO_2CH_3$ | H | H | H | $CH_3$ | $CH_3$ | CH |
| 18 | $CO_2CH_3$ | H | H | H | $CH_3$ | $OCH_3$ | CH |
| 19 | $CO_2CH_3$ | H | H | H | $OCH_3$ | $OCH_3$ | CH |
| 20 | $CO_2CH_3$ | H | H | H | $CH_3$ | $OCH_3$ | N |

TABLE 3 (cont'd)

| Compound No. | $R_1$ | $R_2$ | $R_3$ | $R_4$ | X | Y | Z |
|---|---|---|---|---|---|---|---|
| 21 | $SCH_3$ | H | H | H | $CH_3$ | $CH_3$ | CH |
| 22 | $SCH_3$ | H | H | H | $CH_3$ | $OCH_3$ | CH |
| 23 | $SCH_3$ | H | H | H | $OCH_3$ | $OCH_3$ | CH |
| 24 | $SCH_3$ | H | H | H | $CH_3$ | $OCH_3$ | N |
| 25 | H | H | $CH_3$ | $CH_3$ | $CH_3$ | $CH_3$ | CH |
| 26 | H | H | $CH_3$ | $CH_3$ | $CH_3$ | $OCH_3$ | CH |
| 27 | H | H | $CH_3$ | $CH_3$ | $OCH_3$ | $OCH_3$ | CH |
| 28 | H | H | $CH_3$ | $CH_3$ | $CH_3$ | $OCH_3$ | N |
| 29 | H | Cl | $CH_3$ | $CH_3$ | $CH_3$ | $CH_3$ | CH |
| 30 | H | Cl | $CH_3$ | $CH_3$ | $CH_3$ | $OCH_3$ | CH |
| 31 | H | Cl | $CH_3$ | $CH_3$ | $OCH_3$ | $OCH_3$ | CH |
| 32 | H | Cl | $CH_3$ | $CH_3$ | $CH_3$ | $OCH_3$ | N |
| 33 | Cl | H | $CH_3$ | $CH_3$ | $CH_3$ | $CH_3$ | CH |
| 34 | Cl | H | $CH_3$ | $CH_3$ | $CH_3$ | $OCH_3$ | CH |
| 35 | Cl | H | $CH_3$ | $CH_3$ | $OCH_3$ | $OCH_3$ | CH |
| 36 | Cl | H | $CH_3$ | $CH_3$ | $CH_3$ | $OCH_3$ | N |
| 37 | Br | H | $CH_3$ | $CH_3$ | $CH_3$ | $CH_3$ | CH |
| 38 | Br | H | $CH_3$ | $CH_3$ | $CH_3$ | $OCH_3$ | CH |
| 39 | Br | H | $CH_3$ | $CH_3$ | $OCH_3$ | $OCH_3$ | CH |
| 40 | Br | H | $CH_3$ | $CH_3$ | $CH_3$ | $OCH_3$ | N |
| 41 | $CO_2CH_3$ | H | $CH_3$ | $CH_3$ | $CH_3$ | $CH_3$ | CH |
| 42 | $CO_2CH_3$ | H | $CH_3$ | $CH_3$ | $CH_3$ | $OCH_3$ | CH |
| 43 | $CO_2CH_3$ | H | $CH_3$ | $CH_3$ | $OCH_3$ | $OCH_3$ | CH |
| 44 | $CO_2CH_3$ | H | $CH_3$ | $CH_3$ | $CH_3$ | $OCH_3$ | N |
| 45 | $SCH_3$ | H | $CH_3$ | $CH_3$ | $CH_3$ | $CH_3$ | CH |
| 46 | $SCH_3$ | H | $CH_3$ | $CH_3$ | $CH_3$ | $OCH_3$ | CH |
| 47 | $SCH_3$ | H | $CH_3$ | $CH_3$ | $OCH_3$ | $OCH_3$ | CH |
| 48 | $SCH_3$ | H | $CH_3$ | $CH_3$ | $CH_3$ | $OCH_3$ | N |

## TABLE 4

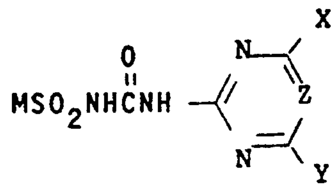

wherein M is $M_4$

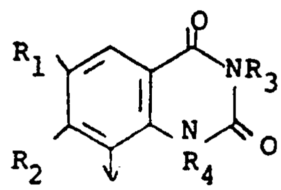

$M_4$

| Compound No. | $R_1$ | $R_2$ | $R_3$ | $R_4$ | X | Y | Z |
|---|---|---|---|---|---|---|---|
| 1 | H | H | H | H | $CH_3$ | $CH_3$ | CH |
| 2 | H | H | H | H | $CH_3$ | $OCH_3$ | CH |
| 3 | H | H | H | H | $OCH_3$ | $OCH_3$ | CH |
| 4 | H | H | H | H | $CH_3$ | $OCH_3$ | N |
| 5 | Cl | H | H | H | $CH_3$ | $CH_3$ | CH |
| 6 | Cl | H | H | H | $CH_3$ | $OCH_3$ | CH |
| 7 | Cl | H | H | H | $OCH_3$ | $OCH_3$ | CH |
| 8 | Cl | H | H | H | $CH_3$ | $OCH_3$ | N |
| 9 | H | Cl | H | H | $CH_3$ | $CH_3$ | CH |
| 10 | H | Cl | H | H | $CH_3$ | $OCH_3$ | CH |
| 11 | H | Cl | H | H | $OCH_3$ | $OCH_3$ | CH |
| 12 | H | Cl | H | H | $CH_3$ | $OCH_3$ | N |
| 13 | H | Br | H | H | $CH_3$ | $CH_3$ | CH |
| 14 | H | Br | H | H | $CH_3$ | $OCH_3$ | CH |
| 15 | H | Br | H | H | $OCH_3$ | $OCH_3$ | CH |
| 16 | H | Br | H | H | $CH_3$ | $OCH_3$ | N |
| 17 | H | $CO_2CH_3$ | H | H | $CH_3$ | $CH_3$ | CH |
| 18 | H | $CO_2CH_3$ | H | H | $CH_3$ | $OCH_3$ | CH |
| 19 | H | $CO_2CH_3$ | H | H | $OCH_3$ | $OCH_3$ | CH |
| 20 | H | $CO_2CH_3$ | H | H | $CH_3$ | $OCH_3$ | N |

TABLE 4 (cont'd)

| Compound No. | $R_1$ | $R_2$ | $R_3$ | $R_4$ | X | Y | Z |
|---|---|---|---|---|---|---|---|
| 21 | H | $SCH_3$ | H | H | $CH_3$ | $CH_3$ | CH |
| 22 | H | $SCH_3$ | H | H | $CH_3$ | $OCH_3$ | CH |
| 23 | H | $SCH_3$ | H | H | $OCH_3$ | $OCH_3$ | CH |
| 24 | H | $SCH_3$ | H | H | $CH_3$ | $OCH_3$ | N |
| 25 | H | H | $CH_3$ | $CH_3$ | $CH_3$ | $CH_3$ | CH |
| 26 | H | H | $CH_3$ | $CH_3$ | $CH_3$ | $OCH_3$ | CH |
| 27 | H | H | $CH_3$ | $CH_3$ | $OCH_3$ | $OCH_3$ | CH |
| 28 | H | H | $CH_3$ | $CH_3$ | $CH_3$ | $OCH_3$ | N |
| 29 | Cl | H | $CH_3$ | $CH_3$ | $CH_3$ | $CH_3$ | CH |
| 30 | Cl | H | $CH_3$ | $CH_3$ | $CH_3$ | $OCH_3$ | CH |
| 31 | Cl | H | $CH_3$ | $CH_3$ | $OCH_3$ | $OCH_3$ | CH |
| 32 | Cl | H | $CH_3$ | $CH_3$ | $CH_3$ | $OCH_3$ | N |
| 33 | H | Cl | $CH_3$ | $CH_3$ | $CH_3$ | $CH_3$ | CH |
| 34 | H | Cl | $CH_3$ | $CH_3$ | $CH_3$ | $OCH_3$ | CH |
| 35 | H | Cl | $CH_3$ | $CH_3$ | $OCH_3$ | $OCH_3$ | CH |
| 36 | H | Cl | $CH_3$ | $CH_3$ | $CH_3$ | $OCH_3$ | N |
| 37 | H | Br | $CH_3$ | $CH_3$ | $CH_3$ | $CH_3$ | CH |
| 38 | H | Br | $CH_3$ | $CH_3$ | $CH_3$ | $OCH_3$ | CH |
| 39 | H | Br | $CH_3$ | $CH_3$ | $OCH_3$ | $OCH_3$ | CH |
| 40 | H | Br | $CH_3$ | $CH_3$ | $CH_3$ | $OCH_3$ | N |
| 41 | H | $CO_2CH_3$ | $CH_3$ | $CH_3$ | $CH_3$ | $CH_3$ | CH |
| 42 | H | $CO_2CH_3$ | $CH_3$ | $CH_3$ | $CH_3$ | $OCH_3$ | CH |
| 43 | H | $CO_2CH_3$ | $CH_3$ | $CH_3$ | $OCH_3$ | $OCH_3$ | CH |
| 44 | H | $CO_2CH_3$ | $CH_3$ | $CH_3$ | $CH_3$ | $OCH_3$ | N |
| 45 | H | $SCH_3$ | $CH_3$ | $CH_3$ | $CH_3$ | $CH_3$ | CH |
| 46 | H | $SCH_3$ | $CH_3$ | $CH_3$ | $CH_3$ | $OCH_3$ | CH |
| 47 | H | $SCH_3$ | $CH_3$ | $CH_3$ | $OCH_3$ | $OCH_3$ | CH |
| 48 | H | $SCH_3$ | $CH_3$ | $CH^3$ | $CH_3$ | $OCH_3$ | N |

TABLE 5

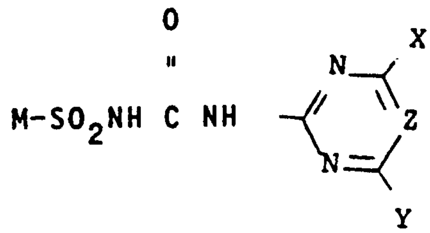

wherein M is $M_5$

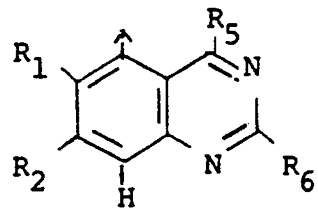

$M_5$

| Compound No. | $R_1$ | $R_2$ | $R_5$ | $R_6$ | X | Y | Z |
|---|---|---|---|---|---|---|---|
| 1 | H | H | H | H | $CH_3$ | $CH_3$ | CH |
| 2 | H | H | H | H | $CH_3$ | $OCH_3$ | CH |
| 3 | H | H | H | H | $OCH_3$ | $OCH_3$ | CH |
| 4 | H | H | H | H | $CH_3$ | $OCH_3$ | N |
| 5 | Cl | H | H | H | $CH_3$ | $CH_3$ | CH |
| 6 | Cl | H | H | H | $CH_3$ | $OCH_3$ | CH |
| 7 | Cl | H | H | H | $OCH_3$ | $OCH_3$ | CH |
| 8 | Cl | H | H | H | $CH_3$ | $OCH_3$ | N |
| 9 | $CO_2CH_3$ | H | H | H | $CH_3$ | $CH_3$ | CH |
| 10 | $CO_2CH_3$ | H | H | H | $CH_3$ | $OCH_3$ | CH |

TABLE 5 (cont'd)

| Compound No. | $R_1$ | $R_2$ | $R_5$ | $R_6$ | X | Y | Z |
|---|---|---|---|---|---|---|---|
| 11 | $CO_2CH_3$ | H | H | H | $OCH_3$ | $OCH_3$ | CH |
| 12 | $CO_2CH_3$ | H | H | H | $CH_3$ | $OCH_3$ | N |
| 13 | $NO_2$ | H | H | H | $CH_3$ | $CH_3$ | CH |
| 14 | $NO_2$ | H | H | H | $CH_3$ | $OCH_3$ | CH |
| 15 | $NO_2$ | H | H | H | $OCH_3$ | $OCH_3$ | CH |
| 16 | $NO_2$ | H | H | H | $CH_3$ | $OCH_3$ | N |
| 17 | $OCH_3$ | H | H | H | $CH_3$ | $CH_3$ | CH |
| 18 | $OCH_3$ | H | H | H | $CH_3$ | $OCH_3$ | CH |
| 19 | $OCH_3$ | H | H | H | $OCH_3$ | $OCH_3$ | CH |
| 20 | $OCH_3$ | H | H | H | $CH_3$ | $OCH_3$ | N |
| 21 | $SCH_3$ | H | H | H | $CH_3$ | $CH_3$ | CH |
| 22 | $SCH_3$ | H | H | H | $CH_3$ | $OCH_3$ | CH |
| 23 | $SCH_3$ | H | H | H | $OCH_3$ | $OCH_3$ | CH |
| 24 | $SCH_3$ | H | H | H | $CH_3$ | $OCH_3$ | N |
| 25 | Br | H | H | H | $CH_3$ | $CH_3$ | CH |
| 26 | Br | H | H | H | $CH_3$ | $OCH_3$ | CH |
| 27 | Br | H | H | H | $OCH_3$ | $OCH_3$ | CH |
| 28 | Br | H | H | H | $CH_3$ | $OCH_3$ | N |
| 29 | H | H | H | $CH_3$ | $CH_3$ | $CH_3$ | CH |
| 30 | H | H | H | $CH_3$ | $CH_3$ | $OCH_3$ | CH |
| 31 | H | H | H | $CH_3$ | $OCH_3$ | $OCH_3$ | CH |
| 32 | H | H | H | $CH_3$ | $CH_3$ | $OCH_3$ | N |
| 33 | Cl | H | H | $CH_3$ | $CH_3$ | $CH_3$ | CH |
| 34 | Cl | H | H | $CH_3$ | $CH_3$ | $OCH_3$ | CH |
| 35 | Cl | H | H | $CH_3$ | $OCH_3$ | $OCH_3$ | CH |
| 36 | Cl | H | H | $CH_3$ | $CH_3$ | $OCH_3$ | N |

TABLE 5 (cont'd)

| Compound No. | $R_1$ | $R_2$ | $R_5$ | $R_6$ | X | Y | Z |
|---|---|---|---|---|---|---|---|
| 37 | $CO_2CH_3$ | H | H | $CH_3$ | $CH_3$ | $CH_3$ | CH |
| 38 | $CO_2CH_3$ | H | H | $CH_3$ | $CH_3$ | $OCH_3$ | CH |
| 39 | $CO_2CH_3$ | H | H | $CH_3$ | $OCH_3$ | $OCH_3$ | CH |
| 40 | $CO_2CH_3$ | H | H | $CH_3$ | $CH_3$ | $OCH_3$ | N |
| 41 | $NO_2$ | H | H | $CH_3$ | $CH_3$ | $CH_3$ | CH |
| 42 | $NO_2$ | H | H | $CH_3$ | $CH_3$ | $OCH_3$ | CH |
| 43 | $NO_2$ | H | H | $CH_3$ | $OCH_3$ | $OCH_3$ | CH |
| 44 | $NO_2$ | H | H | $CH_3$ | $CH_3$ | $OCH_3$ | N |
| 45 | $OCH_3$ | H | H | $CH_3$ | $CH_3$ | $CH_3$ | CH |
| 46 | $OCH_3$ | H | H | $CH_3$ | $CH_3$ | $OCH_3$ | CH |
| 47 | $OCH_3$ | H | H | $CH_3$ | $OCH_3$ | $OCH_3$ | CH |
| 48 | $OCH_3$ | H | H | $CH_3$ | $CH_3$ | $OCH_3$ | N |
| 49 | $SCH_3$ | H | H | $CH_3$ | $CH_3$ | $CH_3$ | CH |
| 50 | $SCH_3$ | H | H | $CH_3$ | $CH_3$ | $OCH_3$ | CH |
| 51 | $SCH_3$ | H | H | $CH_3$ | $OCH_3$ | $OCH_3$ | CH |
| 52 | $SCH_3$ | H | H | $CH_3$ | $CH_3$ | $OCH_3$ | N |
| 53 | Br | H | H | $CH_3$ | $CH_3$ | $CH_3$ | CH |
| 54 | Br | H | H | $CH_3$ | $CH_3$ | $OCH_3$ | CH |
| 55 | Br | H | H | $CH_3$ | $OCH_3$ | $OCH_3$ | CH |
| 56 | Br | H | H | $CH_3$ | $CH_3$ | $OCH_3$ | N |
| 57 | H | H | Cl | $CH_3$ | $CH_3$ | $CH_3$ | CH |
| 58 | H | H | Cl | $CH_3$ | $CH_3$ | $OCH_3$ | CH |
| 59 | H | H | Cl | $CH_3$ | $OCH_3$ | $OCH_3$ | CH |
| 60 | H | H | Cl | $CH_3$ | $CH_3$ | $OCH_3$ | N |

TABLE 5 (cont'd)

| Compound No. | $R_1$ | $R_2$ | $R_5$ | $R_6$ | X | Y | Z |
|---|---|---|---|---|---|---|---|
| 61 | Cl | H | Cl | $CH_3$ | $CH_3$ | $CH_3$ | CH |
| 62 | Cl | H | Cl | $CH_3$ | $CH_3$ | $OCH_3$ | CH |
| 63 | Cl | H | Cl | $CH_3$ | $OCH_3$ | $OCH_3$ | CH |
| 64 | Cl | H | Cl | $CH_3$ | $CH_3$ | $OCH_3$ | N |
| 65 | $CO_2CH_3$ | H | Cl | $CH_3$ | $CH_3$ | $CH_3$ | CH |
| 66 | $CO_2CH_3$ | H | Cl | $CH_3$ | $CH_3$ | $OCH_3$ | CH |
| 67 | $CO_2CH_3$ | H | Cl | $CH_3$ | $CH_3$ | $OCH_3$ | CH |
| 68 | $CO_2CH_3$ | H | Cl | $CH_3$ | $CH_3$ | $OCH_3$ | N |
| 69 | $NO_2$ | H | Cl | $CH_3$ | $CH_3$ | $CH_3$ | CH |
| 70 | $NO_2$ | H | Cl | $CH_3$ | $CH_3$ | $OCH_3$ | CH |
| 71 | $NO_2$ | H | Cl | $CH_3$ | $CH_3$ | $OCH_3$ | CH |
| 72 | $NO_2$ | H | Cl | $CH_3$ | $CH_3$ | $OCH_3$ | N |
| 73 | $OCH_3$ | H | Cl | $CH_3$ | $CH_3$ | $CH_3$ | CH |
| 74 | $OCH_3$ | H | Cl | $CH_3$ | $CH_3$ | $OCH_3$ | CH |
| 75 | $OCH_3$ | H | Cl | $CH_3$ | $OCH_3$ | $OCH_3$ | CH |
| 76 | $OCH_3$ | H | Cl | $CH_3$ | $CH_3$ | $OCH_3$ | N |
| 77 | $SCH_3$ | H | Cl | $CH_3$ | $CH_3$ | $CH_3$ | CH |
| 78 | $SCH_3$ | H | Cl | $CH_3$ | $CH_3$ | $OCH_3$ | CH |
| 79 | $SCH_3$ | H | Cl | $CH_3$ | $OCH_3$ | $OCH_3$ | CH |
| 80 | $SCH_3$ | H | Cl | $CH_3$ | $CH_3$ | $OCH_3$ | N |
| 81 | Br | H | Cl | $CH_3$ | $CH_3$ | $CH_3$ | CH |
| 82 | Br | H | Cl | $CH_3$ | $CH_3$ | $OCH_3$ | CH |
| 83 | Br | H | Cl | $CH_3$ | $OCH_3$ | $OCH_3$ | CH |
| 84 | Br | H | Cl | $CH_3$ | $CH_3$ | $OCH_3$ | N |

TABLE 5 (cont'd)

| Compound No. | $R_1$ | $R_2$ | $R_5$ | $R_6$ | X | Y | Z |
|---|---|---|---|---|---|---|---|
| 85 | H | H | $OCH_3$ | $OCH_3$ | $CH_3$ | $CH_3$ | CH |
| 86 | H | H | $OCH_3$ | $OCH_3$ | $CH_3$ | $OCH_3$ | CH |
| 87 | H | H | $OCH_3$ | $OCH_3$ | $OCH_3$ | $OCH_3$ | CH |
| 88 | H | H | $OCH_3$ | $OCH_3$ | $CH_3$ | $OCH_3$ | N |
| 89 | Cl | H | $OCH_3$ | $OCH_3$ | $CH_3$ | $CH_3$ | CH |
| 90 | Cl | H | $OCH_3$ | $OCH_3$ | $CH_3$ | $OCH_3$ | CH |
| 91 | Cl | H | $OCH_3$ | $OCH_3$ | $OCH_3$ | $OCH_3$ | CH |
| 92 | Cl | H | $OCH_3$ | $OCH_3$ | $CH_3$ | $OCH_3$ | N |
| 93 | $CO_2CH_3$ | H | $OCH_3$ | $OCH_3$ | $CH_3$ | $CH_3$ | CH |
| 94 | $CO_2CH_3$ | H | $OCH_3$ | $OCH_3$ | $CH_3$ | $OCH_3$ | CH |
| 95 | $CO_2CH_3$ | H | $OCH_3$ | $OCH_3$ | $OCH_3$ | $OCH_3$ | CH |
| 96 | $CO_2CH_3$ | H | $OCH_3$ | $OCH_3$ | $CH_3$ | $OCH_3$ | N |
| 97 | $NO_2$ | H | $OCH_3$ | $OCH_3$ | $CH_3$ | $CH_3$ | CH |
| 98 | $NO_2$ | H | $OCH_3$ | $OCH_3$ | $CH_3$ | $OCH_3$ | CH |
| 99 | $NO_2$ | H | $OCH_3$ | $OCH_3$ | $OCH_3$ | $OCH_3$ | CH |
| 100 | $NO_2$ | H | $OCH_3$ | $OCH_3$ | $CH_3$ | $OCH_3$ | N |
| 101 | $OCH_3$ | H | $OCH_3$ | $OCH_3$ | $CH_3$ | $CH_3$ | CH |
| 102 | $OCH_3$ | H | $OCH_3$ | $OCH_3$ | $CH_3$ | $OCH_3$ | CH |
| 103 | $OCH_3$ | H | $OCH_3$ | $OCH_3$ | $OCH_3$ | $OCH_3$ | CH |
| 104 | $OCH_3$ | H | $OCH_3$ | $OCH_3$ | $CH_3$ | $OCH_3$ | N |
| 105 | $SCH_3$ | H | $OCH_3$ | $OCH_3$ | $CH_3$ | $CH_3$ | CH |
| 106 | $SCH_3$ | H | $OCH_3$ | $OCH_3$ | $CH_3$ | $OCH_3$ | CH |
| 107 | $SCH_3$ | H | $OCH_3$ | $OCH_3$ | $OCH_3$ | $OCH_3$ | CH |
| 108 | $SCH_3$ | H | $OCH_3$ | $OCH_3$ | $CH_3$ | $OCH_3$ | N |
| 109 | Br | H | $OCH_3$ | $OCH_3$ | $CH_3$ | $CH_3$ | CH |
| 110 | Br | H | $OCH_3$ | $OCH_3$ | $CH_3$ | $OCH_3$ | CH |
| 111 | Br | H | $OCH_3$ | $OCH_3$ | $OCH_3$ | $OCH_3$ | CH |
| 112 | Br | H | $OCH_3$ | $OCH_3$ | $CH_3$ | $OCH_3$ | N |

TABLE 5 (cont'd)

| Compound No. | $R_1$ | $R_2$ | $R_5$ | $R_6$ | X | Y | Z |
|---|---|---|---|---|---|---|---|
| 113 | H | H | Cl | Cl | $CH_3$ | $CH_3$ | CH |
| 114 | H | H | Cl | Cl | $CH_3$ | $OCH_3$ | CH |
| 115 | H | H | Cl | Cl | $OCH_3$ | $OCH_3$ | CH |
| 116 | H | H | Cl | Cl | $CH_3$ | $OCH_3$ | N |
| 117 | Cl | H | Cl | Cl | $CH_3$ | $CH_3$ | CH |
| 118 | Cl | H | Cl | Cl | $CH_3$ | $OCH_3$ | CH |
| 119 | Cl | H | Cl | Cl | $OCH_3$ | $OCH_3$ | CH |
| 120 | Cl | H | Cl | Cl | $CH_3$ | $OCH_3$ | N |
| 121 | $CO_2CH_3$ | H | Cl | Cl | $CH_3$ | $CH_3$ | CH |
| 122 | $CO_2CH_3$ | H | Cl | Cl | $CH_3$ | $OCH_3$ | CH |
| 123 | $CO_2CH_3$ | H | Cl | Cl | $OCH_3$ | $OCH_3$ | CH |
| 124 | $CO_2CH_3$ | H | Cl | Cl | $CH_3$ | $OCH_3$ | N |
| 125 | $NO_2$ | H | Cl | Cl | $CH_3$ | $CH_3$ | CH |
| 126 | $NO_2$ | H | Cl | Cl | $CH_3$ | $OCH_3$ | CH |
| 127 | $NO_2$ | H | Cl | Cl | $OCH_3$ | $OCH_3$ | CH |
| 128 | $NO_2$ | H | Cl | Cl | $CH_3$ | $OCH_3$ | N |
| 129 | $OCH_3$ | H | Cl | Cl | $CH_3$ | $CH_3$ | CH |
| 130 | $OCH_3$ | H | Cl | Cl | $CH_3$ | $OCH_3$ | CH |
| 131 | $OCH_3$ | H | Cl | Cl | $OCH_3$ | $OCH_3$ | CH |
| 132 | $OCH_3$ | H | Cl | Cl | $CH_3$ | $OCH_3$ | N |
| 133 | $SCH_3$ | H | Cl | Cl | $CH_3$ | $CH_3$ | CH |
| 134 | $SCH_3$ | H | Cl | Cl | $CH_3$ | $OCH_3$ | CH |
| 135 | $SCH_3$ | H | Cl | Cl | $OCH_3$ | $OCH_3$ | CH |
| 136 | $SCH_3$ | H | Cl | Cl | $CH_3$ | $OCH_3$ | N |
| 137 | Br | H | Cl | Cl | $CH_3$ | $CH_3$ | CH |
| 138 | Br | H | Cl | Cl | $CH_3$ | $OCH_3$ | CH |
| 139 | Br | H | Cl | Cl | $OCH_3$ | $OCH_3$ | CH |
| 140 | Br | H | Cl | Cl | $CH_3$ | $OCH_3$ | N |

TABLE 5 (cont'd)

| Compound No. | $R_1$ | $R_2$ | $R_5$ | $R_6$ | X | Y | Z |
|---|---|---|---|---|---|---|---|
| 141 | H | H | $OCH_3$ | $CH_3$ | $CH_3$ | $CH_3$ | CH |
| 142 | H | H | $OCH_3$ | $CH_3$ | $CH_3$ | $OCH_3$ | CH |
| 143 | H | H | $OCH_3$ | $CH_3$ | $OCH_3$ | $OCH_3$ | CH |
| 144 | H | H | $OCH_3$ | $CH_3$ | $CH_3$ | $OCH_3$ | N |
| 145 | Cl | H | $OCH_3$ | $CH_3$ | $CH_3$ | $CH_3$ | CH |
| 146 | Cl | H | $OCH_3$ | $CH_3$ | $CH_3$ | $OCH_3$ | CH |
| 147 | Cl | H | $OCH_3$ | $CH_3$ | $OCH_3$ | $OCH_3$ | CH |
| 148 | Cl | H | $OCH_3$ | $CH_3$ | $CH_3$ | $OCH_3$ | N |
| 149 | $CO_2CH_3$ | H | $OCH_3$ | $CH_3$ | $CH_3$ | $CH_3$ | CH |
| 150 | $CO_2CH_3$ | H | $OCH_3$ | $CH_3$ | $CH_3$ | $OCH_3$ | CH |
| 151 | $CO_2CH_3$ | H | $OCH_3$ | $CH_3$ | $OCH_3$ | $OCH_3$ | CH |
| 152 | $CO_2CH_3$ | H | $OCH_3$ | $CH_3$ | $CH_3$ | $CH_3$ | N |
| 153 | $NO_2$ | H | $OCH_3$ | $CH_3$ | $CH_3$ | $CH_3$ | CH |
| 154 | $NO_2$ | H | $OCH_3$ | $CH_3$ | $CH_3$ | $OCH_3$ | CH |
| 155 | $NO_2$ | H | $OCH_3$ | $CH_3$ | $OCH_3$ | $OCH_3$ | CH |
| 156 | $NO_2$ | H | $OCH_3$ | $CH_3$ | $CH_3$ | $OCH_3$ | N |
| 157 | $OCH_3$ | H | $OCH_3$ | $CH_3$ | $CH_3$ | $CH_3$ | CH |
| 158 | $OCH_3$ | H | $OCH_3$ | $CH_3$ | $CH_3$ | $OCH_3$ | CH |
| 159 | $OCH_3$ | H | $OCH_3$ | $CH_3$ | $OCH_3$ | $OCH_3$ | CH |
| 160 | $OCH_3$ | H | $OCH_3$ | $CH_3$ | $CH_3$ | $OCH_3$ | N |
| 161 | $SCH_3$ | H | $OCH_3$ | $CH_3$ | $CH_3$ | $CH_3$ | CH |
| 162 | $SCH_3$ | H | $OCH_3$ | $CH_3$ | $CH_3$ | $OCH_3$ | CH |
| 163 | $SCH_3$ | H | $OCH_3$ | $CH_3$ | $OCH_3$ | $OCH_3$ | CH |
| 164 | $SCH_3$ | H | $OCH_3$ | $CH_3$ | $CH_3$ | $OCH_3$ | N |
| 165 | Br | H | $OCH_3$ | $CH_3$ | $CH_3$ | $CH_3$ | CH |
| 166 | Br | H | $OCH_3$ | $CH_3$ | $CH_3$ | $OCH_3$ | CH |
| 167 | Br | H | $OCH_3$ | $CH_3$ | $OCH_3$ | $OCH_3$ | CH |
| 168 | Br | H | $OCH_3$ | $CH_3$ | $CH_3$ | $OCH_3$ | N |

## TABLE 6

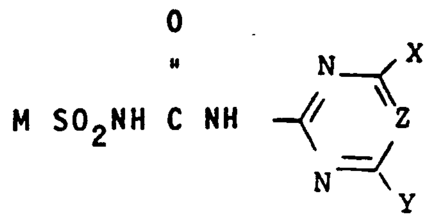

wherein M is $M_6$

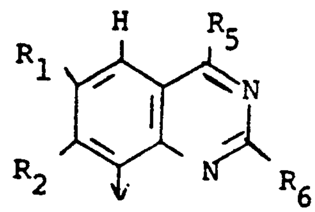

$M_6$

| Compound No. | $R_1$ | $R_2$ | $R_5$ | $R_6$ | X | Y | Z |
|---|---|---|---|---|---|---|---|
| 1 | H | H | H | H | $CH_3$ | $CH_3$ | CH |
| 2 | H | H | H | H | $CH_3$ | $OCH_3$ | CH |
| 3 | H | H | H | H | $OCH_3$ | $OCH_3$ | CH |
| 4 | H | H | H | H | $CH_3$ | $OCH_3$ | N |
| 5 | H | Cl | H | H | $CH_3$ | $CH_3$ | CH |
| 6 | H | Cl | H | H | $CH_3$ | $OCH_3$ | CH |
| 7 | H | Cl | H | H | $OCH_3$ | $OCH_3$ | CH |
| 8 | H | Cl | H | H | $CH_3$ | $OCH_3$ | N |
| 9 | H | $CO_2CH_3$ | H | H | $CH_3$ | $CH_3$ | CH |
| 10 | H | $CO_2CH_3$ | H | H | $CH_3$ | $OCH_3$ | CH |

TABLE 6 (cont'd)

| Compound No. | $R_1$ | $R_2$ | $R_5$ | $R_6$ | X | Y | Z |
|---|---|---|---|---|---|---|---|
| 11 | H | $CO_2CH_3$ | H | H | $OCH_3$ | $OCH_3$ | CH |
| 12 | H | $CO_2CH_3$ | H | H | $CH_3$ | $OCH_3$ | N |
| 13 | H | $NO_2$ | H | H | $CH_3$ | $CH_3$ | CH |
| 14 | H | $NO_2$ | H | H | $CH_3$ | $CH_3$ | CH |
| 15 | H | $NO_2$ | H | H | $OCH_3$ | $OCH_3$ | CH |
| 16 | H | $NO_2$ | H | H | $CH_3$ | $OCH_3$ | N |
| 17 | H | $OCH_3$ | H | H | $CH_3$ | $CH_3$ | CH |
| 18 | H | $OCH_3$ | H | H | $CH_3$ | $OCH_3$ | CH |
| 19 | H | $OCH_3$ | H | H | $OCH_3$ | $OCH_3$ | CH |
| 20 | H | $OCH_3$ | H | H | $CH_3$ | $OCH_3$ | N |
| 21 | H | $SCH_3$ | H | H | $CH_3$ | $CH_3$ | CH |
| 22 | H | $SCH_3$ | H | H | $CH_3$ | $OCH_3$ | CH |
| 23 | H | $SCH_3$ | H | H | $OCH_3$ | $OCH_3$ | CH |
| 24 | H | $SCH_3$ | H | H | $CH_3$ | $OCH_3$ | N |
| 25 | H | Br | H | H | $CH_3$ | $CH_3$ | CH |
| 26 | H | Br | H | H | $CH_3$ | $OCH_3$ | CH |
| 27 | H | Br | H | H | $OCH_3$ | $OCH_3$ | CH |
| 28 | H | Br | H | H | $CH_3$ | $OCH_3$ | N |
| 29 | H | H | H | $CH_3$ | $CH_3$ | $CH_3$ | CH |
| 30 | H | H | H | $CH_3$ | $CH_3$ | $OCH_3$ | CH |
| 31 | H | H | H | $CH_3$ | $OCH_3$ | $OCH_3$ | CH |
| 32 | H | H | H | $CH_3$ | $CH_3$ | $OCH_3$ | N |
| 33 | H | Cl | H | $CH_3$ | $CH_3$ | $CH_3$ | CH |
| 34 | H | Cl | H | $CH_3$ | $CH_3$ | $OCH_3$ | CH |
| 35 | H | Cl | H | $CH_3$ | $OCH_3$ | $OCH_3$ | CH |
| 36 | H | Cl | H | $CH_3$ | $CH_3$ | $OCH_3$ | N |
| 37 | H | $CO_2CH_3$ | H | $CH_3$ | $CH_3$ | $CH_3$ | CH |
| 38 | H | $CO_2CH_3$ | H | $CH_3$ | $CH_3$ | $OCH_3$ | CH |
| 39 | H | $CO_2CH_3$ | H | $CH_3$ | $OCH_3$ | $OCH_3$ | CH |

TABLE 6 (cont'd)

| Compound No. | $R_1$ | $R_2$ | $R_5$ | $R_6$ | X | Y | Z |
|---|---|---|---|---|---|---|---|
| 40 | H | $CO_2CH_3$ | H | $CH_3$ | $CH_3$ | $OCH_3$ | N |
| 41 | H | $NO_2$ | H | $CH_3$ | $CH_3$ | $CH_3$ | CH |
| 42 | H | $NO_2$ | H | $CH_3$ | $CH_3$ | $OCH_3$ | CH |
| 43 | H | $NO_2$ | H | $CH_3$ | $OCH_3$ | $OCH_3$ | CH |
| 44 | H | $NO_2$ | H | $CH_3$ | $CH_3$ | $OCH_3$ | N |
| 45 | H | $OCH_3$ | H | $CH_3$ | $CH_3$ | $CH_3$ | CH |
| 46 | H | $OCH_3$ | H | $CH_3$ | $CH_3$ | $OCH_3$ | CH |
| 47 | H | $OCH_3$ | H | $CH_3$ | $OCH_3$ | $OCH_3$ | CH |
| 48 | H | $OCH_3$ | H | $CH_3$ | $CH_3$ | $OCH_3$ | N |
| 49 | H | $SCH_3$ | H | $CH_3$ | $CH_3$ | $CH_3$ | CH |
| 50 | H | $SCH_3$ | H | $CH_3$ | $CH_3$ | $OCH_3$ | CH |
| 51 | H | $SCH_3$ | H | $CH_3$ | $OCH_3$ | $OCH_3$ | CH |
| 52 | H | $SCH_3$ | H | $CH_3$ | $CH_3$ | $OCH_3$ | N |
| 53 | H | Br | H | $CH_3$ | $CH_3$ | $CH_3$ | CH |
| 54 | H | Br | H | $CH_3$ | $CH_3$ | $OCH_3$ | CH |
| 55 | H | Br | H | $CH_3$ | $OCH_3$ | $OCH_3$ | CH |
| 56 | H | Br | H | $CH_3$ | $CH_3$ | $OCH_3$ | N |
| 57 | H | H | Cl | $CH_3$ | $CH_3$ | $CH_3$ | CH |
| 58 | H | H | Cl | $CH_3$ | $CH_3$ | $OCH_3$ | CH |
| 59 | H | H | Cl | $CH_3$ | $OCH_3$ | $OCH_3$ | CH |
| 60 | H | H | Cl | $CH_3$ | $CH_3$ | $OCH_3$ | N |
| 61 | H | Cl | Cl | $CH_3$ | $CH_3$ | $CH_3$ | CH |
| 62 | H | Cl | Cl | $CH_3$ | $CH_3$ | $OCH_3$ | CH |
| 63 | H | Cl | Cl | $CH_3$ | $OCH_3$ | $OCH_3$ | CH |
| 64 | H | Cl | Cl | $CH_3$ | $CH_3$ | $OCH_3$ | N |
| 65 | H | $CO_2CH_3$ | Cl | $CH_3$ | $CH_3$ | $CH_3$ | CH |
| 66 | H | $CO_2CH_3$ | Cl | $CH_3$ | $CH_3$ | $OCH_3$ | CH |

TABLE 6 (cont'd)

| Compound No. | $R_1$ | $R_2$ | $R_5$ | $R_6$ | X | Y | Z |
|---|---|---|---|---|---|---|---|
| 67 | H | $CO_2CH_3$ | Cl | $CH_3$ | $OCH_3$ | $OCH_3$ | CH |
| 68 | H | $CO_2CH_3$ | Cl | $CH_3$ | $CH_3$ | $OCH_3$ | N |
| 69 | H | $NO_2$ | Cl | $CH_3$ | $CH_3$ | $CH_3$ | CH |
| 70 | H | $NO_2$ | Cl | $CH_3$ | $CH_3$ | $OCH_3$ | CH |
| 71 | H | $NO_2$ | Cl | $CH_3$ | $OCH_3$ | $OCH_3$ | CH |
| 72 | H | $NO_2$ | Cl | $CH_3$ | $CH_3$ | $OCH_3$ | N |
| 73 | H | $OCH_3$ | Cl | $CH_3$ | $CH_3$ | $CH_3$ | CH |
| 74 | H | $OCH_3$ | Cl | $CH_3$ | $CH_3$ | $OCH_3$ | CH |
| 75 | H | $OCH_3$ | Cl | $CH_3$ | $OCH_3$ | $OCH_3$ | CH |
| 76 | H | $OCH_3$ | Cl | $CH_3$ | $CH_3$ | $OCH_3$ | N |
| 77 | H | $SCH_3$ | Cl | $CH_3$ | $CH_3$ | $CH_3$ | CH |
| 78 | H | $SCH_3$ | Cl | $CH_3$ | $CH_3$ | $OCH_3$ | CH |
| 79 | H | $SCH_3$ | Cl | $CH_3$ | $OCH_3$ | $OCH_3$ | CH |
| 80 | H | $SCH_3$ | Cl | $CH_3$ | $CH_3$ | $OCH_3$ | N |
| 81 | H | Br | Cl | $CH_3$ | $CH_3$ | $CH_3$ | CH |
| 82 | H | Br | Cl | $CH_3$ | $CH_3$ | $OCH_3$ | CH |
| 83 | H | Br | Cl | $CH_3$ | $OCH_3$ | $OCH_3$ | CH |
| 84 | H | Br | Cl | $CH_3$ | $CH_3$ | $OCH_3$ | N |
| 85 | H | H | $OCH_3$ | $OCH_3$ | $CH_3$ | $CH_3$ | CH |
| 86 | H | H | $OCH_3$ | $OCH_3$ | $CH_3$ | $OCH_3$ | CH |
| 87 | H | H | $OCH_3$ | $OCH_3$ | $OCH_3$ | $OCH_3$ | CH |
| 88 | H | H | $OCH_3$ | $OCH_3$ | $CH_3$ | $OCH_3$ | N |
| 89 | H | Cl | $OCH_3$ | $OCH_3$ | $CH_3$ | $CH_3$ | CH |
| 90 | H | Cl | $OCH_3$ | $OCH_3$ | $CH_3$ | $OCH_3$ | CH |
| 91 | H | Cl | $OCH_3$ | $OCH_3$ | $OCH_3$ | $OCH_3$ | CH |
| 92 | H | Cl | $OCH_3$ | $OCH_3$ | $CH_3$ | $OCH_3$ | N |
| 93 | H | $CO_2CH_3$ | $OCH_3$ | $OCH_3$ | $CH_3$ | $CH_3$ | CH |
| 94 | H | $CO_2CH_3$ | $OCH_3$ | $OCH_3$ | $CH_3$ | $OCH_3$ | CH |

TABLE 6 (cont'd)

| Compound No. | $R_1$ | $R_2$ | $R_5$ | $R_6$ | X | Y | Z |
|---|---|---|---|---|---|---|---|
| 95 | H | $CO_2CH_3$ | $OCH_3$ | $OCH_3$ | $OCH_3$ | $OCH_3$ | CH |
| 96 | H | $CO_2CH_3$ | $OCH_3$ | $OCH_3$ | $CH_3$ | $OCH_3$ | N |
| 97 | H | $NO_2$ | $OCH_3$ | $OCH_3$ | $CH_3$ | $CH_3$ | CH |
| 98 | H | $NO_2$ | $OCH_3$ | $OCH_3$ | $CH_3$ | $OCH_3$ | CH |
| 99 | H | $NO_2$ | $OCH_3$ | $OCH_3$ | $OCH_3$ | $OCH_3$ | CH |
| 100 | H | $NO_2$ | $OCH_3$ | $OCH_3$ | $CH_3$ | $OCH_3$ | N |
| 101 | H | $OCH_3$ | $OCH_3$ | $OCH_3$ | $CH_3$ | $CH_3$ | CH |
| 102 | H | $OCH_3$ | $OCH_3$ | $OCH_3$ | $CH_3$ | $OCH_3$ | CH |
| 103 | H | $OCH_3$ | $OCH_3$ | $OCH_3$ | $OCH_3$ | $OCH_3$ | CH |
| 104 | H | $OCH_3$ | $OCH_3$ | $OCH_3$ | $CH_3$ | $OCH_3$ | N |
| 105 | H | $SCH_3$ | $OCH_3$ | $OCH_3$ | $CH_3$ | $CH_3$ | CH |
| 106 | H | $SCH_3$ | $OCH_3$ | $OCH_3$ | $CH_3$ | $OCH_3$ | CH |
| 107 | H | $SCH_3$ | $OCH_3$ | $OCH_3$ | $OCH_3$ | $OCH_3$ | CH |
| 108 | H | $SCH_3$ | $OCH_3$ | $OCH_3$ | $CH_3$ | $OCH_3$ | N |
| 109 | H | Br | $OCH_3$ | $OCH_3$ | $CH_3$ | $CH_3$ | CH |
| 110 | H | Br | $OCH_3$ | $OCH_3$ | $CH_3$ | $OCH_3$ | CH |
| 111 | H | Br | $OCH_3$ | $OCH_3$ | $OCH_3$ | $OCH_3$ | CH |
| 112 | H | Br | $OCH_3$ | $OCH_3$ | $CH_3$ | $OCH_3$ | N |
| 113 | H | H | Cl | Cl | $CH_3$ | $CH_3$ | CH |
| 114 | H | H | Cl | Cl | $CH_3$ | $OCH_3$ | CH |
| 115 | H | H | Cl | Cl | $OCH_3$ | $OCH_3$ | CH |
| 116 | H | H | Cl | Cl | $CH_3$ | $OCH_3$ | N |
| 117 | Cl | H | Cl | Cl | $CH_3$ | $CH_3$ | CH |
| 118 | Cl | H | Cl | Cl | $CH_3$ | $OCH_3$ | CH |
| 119 | Cl | H | Cl | Cl | $OCH_3$ | $OCH_3$ | CH |
| 120 | Cl | H | Cl | Cl | $CH_3$ | $OCH_3$ | N |
| 121 | $CO_2CH_3$ | H | Cl | Cl | $CH_3$ | $CH_3$ | CH |

TABLE 6 (cont'd)

| Compound No. | $R_1$ | $R_2$ | $R_5$ | $R_6$ | X | Y | Z |
|---|---|---|---|---|---|---|---|
| 122 | $CO_2CH_3$ | H | Cl | Cl | $CH_3$ | $OCH_3$ | CH |
| 123 | $CO_2CH_3$ | H | Cl | Cl | $OCH_3$ | $OCH_3$ | CH |
| 124 | $CO_2CH_3$ | H | Cl | Cl | $CH_3$ | $OCH_3$ | N |
| 125 | $NO_2$ | H | Cl | Cl | $CH_3$ | $CH_3$ | CH |
| 126 | $NO_2$ | H | Cl | Cl | $CH_3$ | $OCH_3$ | CH |
| 127 | $NO_2$ | H | Cl | Cl | $OCH_3$ | $OCH_3$ | CH |
| 128 | $NO_2$ | H | Cl | Cl | $CH_3$ | $OCH_3$ | N |
| 129 | H | $OCH_3$ | Cl | Cl | $CH_3$ | $CH_3$ | CH |
| 130 | H | $OCH_3$ | Cl | Cl | $CH_3$ | $OCH_3$ | CH |
| 131 | H | $OCH_3$ | Cl | Cl | $OCH_3$ | $OCH_3$ | CH |
| 132 | H | $OCH_3$ | Cl | Cl | $CH_3$ | $OCH_3$ | N |
| 133 | H | $SCH_3$ | Cl | Cl | $CH_3$ | $CH_3$ | CH |
| 134 | H | $SCH_3$ | Cl | Cl | $CH_3$ | $OCH_3$ | CH |
| 135 | H | $SCH_3$ | Cl | Cl | $OCH_3$ | $OCH_3$ | CH |
| 136 | H | $SCH_3$ | Cl | Cl | $CH_3$ | $OCH_3$ | N |
| 137 | H | Br | Cl | Cl | $CH_3$ | $CH_3$ | CH |
| 138 | H | Br | Cl | Cl | $CH_3$ | $OCH_3$ | CH |
| 139 | H | Br | Cl | Cl | $OCH_3$ | $OCH_3$ | CH |
| 140 | H | Br | Cl | Cl | $CH_3$ | $OCH_3$ | N |
| 141 | H | H | $OCH_3$ | $CH_3$ | $CH_3$ | $CH_3$ | CH |
| 142 | H | H | $OCH_3$ | $CH_3$ | $CH_3$ | $OCH_3$ | CH |
| 143 | H | H | $OCH_3$ | $CH_3$ | $OCH_3$ | $OCH_3$ | CH |
| 144 | H | H | $OCH_3$ | $CH_3$ | $CH_3$ | $OCH_3$ | N |
| 145 | H | Cl | $OCH_3$ | $CH_3$ | $CH_3$ | $CH_3$ | CH |
| 146 | H | Cl | $OCH_3$ | $CH_3$ | $CH_3$ | $OCH_3$ | CH |
| 147 | H | Cl | $OCH_3$ | $CH_3$ | $OCH_3$ | $OCH_3$ | CH |
| 148 | H | Cl | $OCH_3$ | $CH_3$ | $CH_3$ | $OCH_3$ | N |
| 149 | H | $CO_2CH_3$ | $OCH_3$ | $CH_3$ | $CH_3$ | $CH_3$ | CH |
| 150 | H | $CO_2CH_3$ | $OCH_3$ | $CH_3$ | $CH_3$ | $OCH_3$ | CH |

TABLE 6 (cont'd)

| Compound No. | $R_1$ | $R_2$ | $R_5$ | $R_6$ | X | Y | Z |
|---|---|---|---|---|---|---|---|
| 151 | H | $CO_2CH_3$ | $OCH_3$ | $CH_3$ | $OCH_3$ | $OCH_3$ | CH |
| 152 | H | $CO_2CH_3$ | $OCH_3$ | $CH_3$ | $CH_3$ | $OCH_3$ | N |
| 153 | H | $NO_2$ | $OCH_3$ | $CH_3$ | $CH_3$ | $CH_3$ | CH |
| 154 | H | $NO_2$ | $OCH_3$ | $CH_3$ | $CH_3$ | $OCH_3$ | CH |
| 155 | H | $NO_2$ | $OCH_3$ | $CH_3$ | $OCH_3$ | $OCH_3$ | CH |
| 156 | H | $NO_2$ | $OCH_3$ | $CH_3$ | $CH_3$ | $OCH_3$ | N |
| 157 | H | $OCH_3$ | $OCH_3$ | $CH_3$ | $CH_3$ | $CH_3$ | CH |
| 158 | H | $OCH_3$ | $OCH_3$ | $CH_3$ | $CH_3$ | $OCH_3$ | CH |
| 159 | H | $OCH_3$ | $OCH_3$ | $CH_3$ | $OCH_3$ | $OCH_3$ | CH |
| 160 | H | $OCH_3$ | $OCH_3$ | $CH_3$ | $CH_3$ | $OCH_3$ | N |
| 161 | H | $SCH_3$ | $OCH_3$ | $CH_3$ | $CH_3$ | $CH_3$ | CH |
| 162 | H | $SCH_3$ | $OCH_3$ | $CH_3$ | $CH_3$ | $OCH_3$ | CH |
| 163 | H | $SCH_3$ | $OCH_3$ | $CH_3$ | $OCH_3$ | $OCH_3$ | CH |
| 164 | H | $SCH_3$ | $OCH_3$ | $CH_3$ | $CH_3$ | $OCH_3$ | N |
| 165 | H | Br | $OCH_3$ | $CH_3$ | $CH_3$ | $CH_3$ | CH |
| 166 | H | Br | $OCH_3$ | $CH_3$ | $CH_3$ | $OCH_3$ | CH |
| 167 | H | Br | $OCH_3$ | $CH_3$ | $OCH_3$ | $OCH_3$ | CH |
| 168 | H | Br | $OCH_3$ | $CH_3$ | $CH_3$ | $OCH_3$ | N |

The compounds of the invention may be prepared by a variety of methods and in a further aspect the invention provides methods for preparation of compounds of formula I. Conveniently the preparation of the compounds of the invention can be considered in three parts. Part A involves the preparation of fused phenylsulfonamides of the formula II

$QSO_2NH_2$ II

wherein Q is as defined for formula I and wherein $R_1$ through to $R_7$, $W_1$ and $W_2$ are as defined for formula I. The sulfonamides of formula II can be prepared in a variety of ways including the general methods outlined below.

(a) The requisite sulfonamides II can be synthesised from the corresponding sulfonyl chlorides III as outlined in Equation I

Equation I

$$\underset{III}{QSO_2Cl} \xrightarrow[NH_4OH]{NH_3 \text{ or}} \underset{II}{QSO_2NH_2}$$

wherein

Q is as previously defined.

The reaction shown in Equation 1 is most conveniently carried out by adding either excess anhydrous ammonia or concentrated ammonium hydroxide to a solution of the sulfonyl chloride III, in a suitable solvent such as tetrahydrofuran or diethyl ether at -30°C to 0°C.

Sulfonyl chlorides a Formula III can be 20 prepared by one or more of the methods shown below in Equations 2, 3 or 4.

Equation 2 depicts the oxidative chlorination of thiols or thioethers of Formula IV.

Equation 2

$$\underset{IV}{QSR_9} \xrightarrow[H_2O]{Cl_2} \underset{III}{QSO_2Cl}$$

wherein

$R_9$ is hydrogen or benzyl.

The reaction shown in Equation 2 is best effected in a suitable solvent such as chloroform or methylene chloride; in some cases, it is advantageous to use acetic acid as solvent. The reaction is carried out in the presence of at least 2.5 equivalents of water and three equivalents of chlorine gas at temperatures between 0° and 30°C for one to five hours. The products are most conveniently isolated by filtration and are normally carried on to the next step without purification.

Another method for the preparation of sulfonyl chlorides III is outlined in Equation 3 and involves the reaction of unsubstituted compounds of Formula V with chlorosulfonic acid.

Equation 3

$$\underset{V}{Q{-}H} \xrightarrow{ClSO_3H} \underset{III}{QSO_2Cl}$$

The reaction in Equation 3 is best carried out by addition of the compounds V to excess chlorosulfonic acid. The reaction mixture is then typically heated to 100-150°C for one to five hours, cooled to 25°C, and cautiously poured onto crushed ice. The desired sulfonyl chlorides III are generally isolated by filtration.

A third procedure for the synthesis of sulfonyl chlorides of Formula III involves the diazotization of aniline derivatives VI and coupling with sulfur dioxide in the presence of cupric chloride as shown in Equation 4 [J.Org. Chem; 25, 1824 (1960)].

Equation 4

$$Q{-}NH_2 \xrightarrow[2)\ SO_2,\ CuCl_2]{1)\ NaNO_2,\ HCl} QSO_2Cl$$

VI III

The requisite aniline derivatives of Formula VI can be prepared in a straightforward manner by reduction of the corresponding nitro compounds of Formula VII as shown in Equation 5.

Equation 5

$$Q{-}NO_2 \xrightarrow{[H]} Q{-}NH_2$$

VII VI

There exists a wide variety of methods for effecting the reduction of aromatic nitro derivatives to the corresponding anilines. One of the more common procedures involves treating the nitro compounds of Formula VII with a slight excess of stannous chloride dihydrate is concentrated hydrochloric acid or ethanol solution at temperatures between 25° and 80°C. Alternatively, reduction can be accomplished with iron powder in glacial acetic acid solution as described by Hazlet and Dornfeld, J. Am. Chem. Soc, 66, 1781 (1944). For a general review, see Groggins, "Unit Processes in Organic Synthesis", McGraw-Hill Book Co., New York, 1947, pp. 73-128.

The appropriate nitro intermediates VII can also be obtained by nitration of the parent quinazoline V of alternatively from an appropriately substituted nitroanthranilic acid VIIIa as outlined in equation 6(a).

Equation 6

(a)

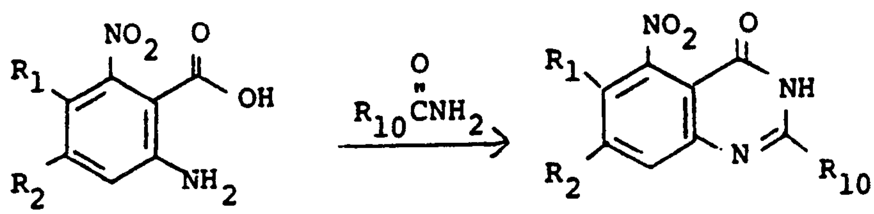

$R_{10}$ = H, alkyl or aryl

VIIIa VIIa

(b)

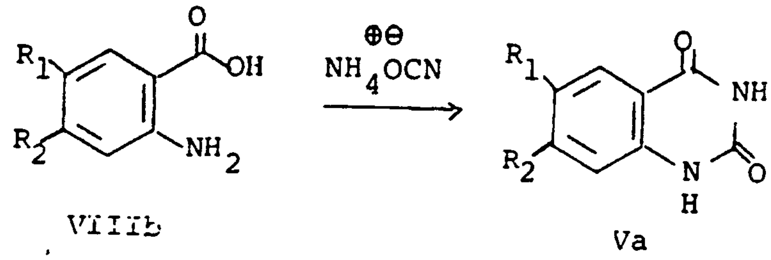


VIIIb

Va

(c)

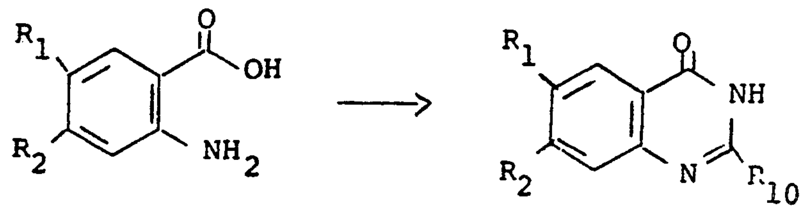

VIIIc

Vb

Many of the requisite intermediates mentioned above are either known in the literature or can be synthesized by methods known to one who is skilled in the art.

The reactions outlined in equation 6 are well precedented in the literature. Quinazolines can be prepared from appropriate anthranillic acid derivatives VIII according to the procedures of Baker, B.R. et al J.Org.Chem. (1952) 164 and Leonard, N.J. et al J.Org.Chem. (1975), 40, 363. For a comprehensive compilation of references dealing with the synthesis and functionalisation of quinazolines, see W.L.F. Armarego in "Hetercyclic Compounds", Part 1, Quinazolines, ed. D.J. Brown, John Wiley and Sons (Interscience), New York, 1967.

Another approach to the desired thioether IV involves nucleophilic displacement of leaving group L from IX as outlined in equation 7.

## Equation 7

$$QL \xrightarrow{R_9SH} QSR_9$$

IX IV

Examples of sulfonamide II may be prepared from IIa as outlined in Equation 8, (where Q = Q5 and Q6), utilising methodology well known in the literature.

## Equation 8

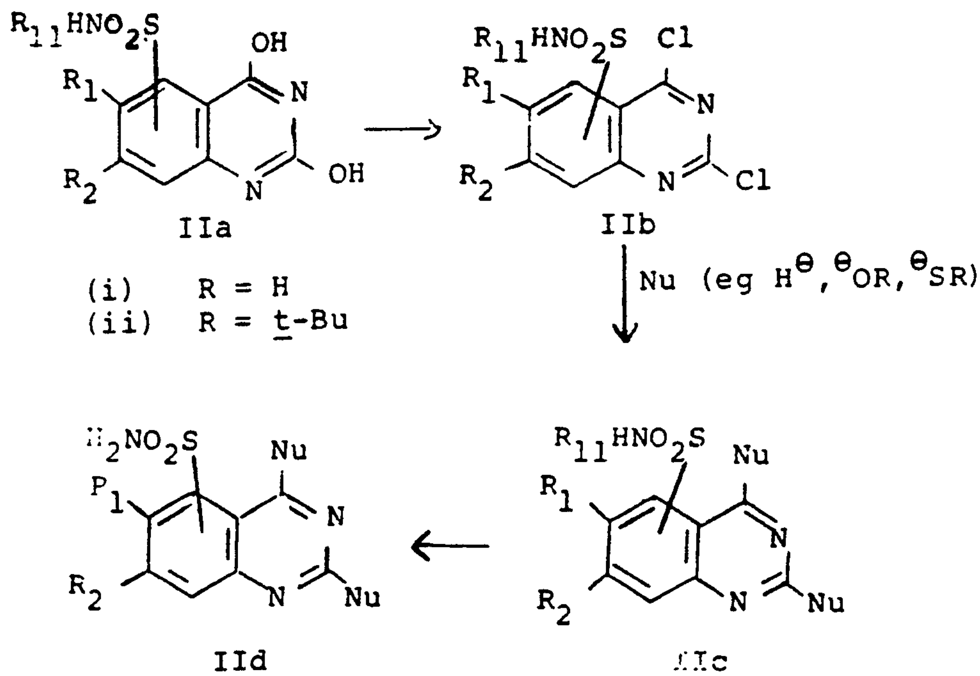


IIa IIb IIc IId

In equation 8 above, group $R_{11}$ is either hydrogen or a tertiary butyl protecting group; the latter protecting group can be removed readily with an acid e.g. trifluoroacetic acid.

Part B of the preparation of the compounds of the invention involves the preparation of the various nitrogen-containing heterocycles A-1 to A-7.

The heterocyclic amines of Formula X can be prepared by methods known in the literature, or simple modifications thereof, by one skilled in the art.

$\underset{R}{\underset{|}{HN}}-A$    X

For a review of the synthesis and reactions of 2- amino- and 2-methylaminopyrimidines (X, A = A-1, Z = CH) see The Chemistry of Heterocyclic Compounds. Vol. 16, Wiley-Interscience, New York (1962). For a review of the synthesis and reactions of 2-amino- and 2-methylamino-s-triazines (X, A = A-1, Z = N) see The Chemistry of Heterocyclic Compounds. Vol. 13, Wiley-Interscience, New York (1959), and F. C. Schaefer and K. R. Huffman, J. Org. Chem., 28, 1812 (1963). EP-A No. 84,224 and W. Braker et al., J. Amer Chem. Soc., 69, 3072 (1947) describe methods for preparing aminopyrimidines and triazines substituted by acetal groups such as dialkoxymethyl or 1,3-dioxolan-2-yl. U.S. Patent 4,515,626 describes methods for the synthesis of cyclopropyl-pyrimidines and triazines substituted by such groups as alkyl, haloalkyl, alkoxy, haloalkoxy, alkylamino, dialkylamino or alkoxyalkyl.

The 5,6-dihydrofuro[2.3-d]pyrimidine-2-amines, the cyclopenta[d]pyrimidines-2-amines (formula X wherein A is A-2) and the 6,7-dihydro-5H-pyrano[2,3-d]pyrimidin-2-amines (formula X wherein A is A-3) can be prepared as taught in U.S. 4,339,267. The furo[2,3-d]pyrimidin-2-amines (X, A is A-4) are described in U.S. 4,487,626.

Compounds of formula X where A is A-5, are described in EPA- 0 073 562. Compounds of Formula X where A is A-6, are described in U.S. 4,496,392.

The amines of Formula X where A is A-7 can be prepared by methods taught in EP-A- 0 125 864 (published 21/11/84) or by suitable modifications that would be obvious to one skilled in the art.

Part C of the preparation of the compounds of the invention (formula I) involves the coupling of the sulfonamides of formula II with the heterocyclic amines of formula X. The compounds of formula I can be prepared by one or more of the methods described below.

a) Many of the compounds of formula I can be prepared by reacting a sulfonylisocyanate or a sulfonylisothiocyanate of formula XI with a heterocyclic amine of formula X.

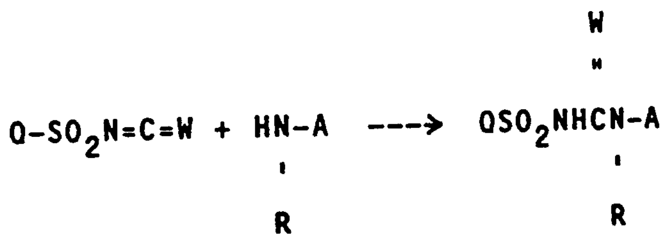

XI X I

wherein Q is as defined for formula I
and W, $W_1$, $W_2$, R through to $R_8$ and A are as previously defined.

The reaction is carried out at 25° to 100°C in an inert, aprotic solvent such as methylene chloride or xylene for 0.5 to 24 hours as taught in U.S. Patent 4,127,405

The intermediate sulfonylisocyanates (formula XI wherein W=O) and isothiocyanates (formula XI wherein W=S) may be prepared by a variety of methods which are well known in the art and are described for example in EP-A- 0 212 779 Patent Application 212,779 and the references cited therein.

b) Many of the compounds of formula I, where W is oxygen, can be prepared by reacting a phenyl carbamate of formula XII with a suitable amine of formula X.

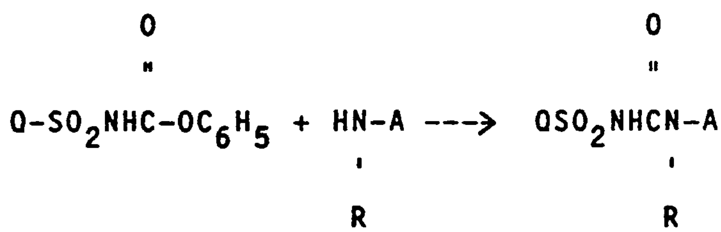

XII X Ia

The reaction is carried out at 0° to 100°C in a solvent such as dioxane or N,N-dimethyformamide for 0.5 to 24 hours. The required carbamates XII are prepared by reacting the corresponding sulfonamides II with diphenylcarbonate in the presence of a strong base.

c) Compounds of formula Ia can also be made by reacting a heterocyclic carbamate of formula XIII with a suitable sulfonamide of formula II.

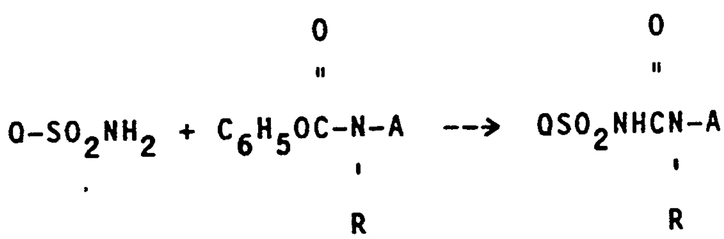

II XIII Ia

The reaction is carried out at 0° to 100°C in a solvent such as acetonitrile, dioxane or N,N-dimethylformamide in the presence of a non-nucleophilic base such as DBU for 0.2 to 24 hours. The required phenylcarbamates XIII are prepared by reacting the corresponding heterocyclic amines X with diphenylcarbonate or phenylchloroformate in the presence of a strong base.
Alternatively, the methylcarbamate derivative of the heterocyclic amine X may be used.

d) Some of the compounds of the invention of formula Ib can be prepared by reacting a sulfonamide

II with a heterocyclic isocyanate or isothiocyanate of formula XIV.

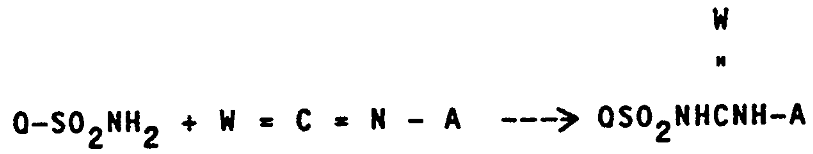

II XIV Ib

The reaction is carried out at 25° to 80°C in an inert, aprotic solvent such as acetone or acetonitrile in the presence of a base such as potassium carbonate for 0.5 to 24 hours. The required heterocyclic isocyanates and iso-thiocyanates XIV are prepared from the corresponding amines $H_2NA$ which would be known to one skilled in the art as taught in EP-A- 0 035 893.

In each of parts b), c) and d) above the groups Q, W, A and R are as previously described.

Certain of the intermediate compounds of formulae II, III IV, V, VI, VII and VIII are novel compounds and therefore in further embodiments the invention provides novel compounds of formulae II, III, IV, V, VI, VII and VIII and processes for the preparation thereof.

Agriculturally suitable salts of compounds of Formula I are also useful herbicides and can be prepared in a number of ways known to the art. For example, metal salts can be made by contacting compounds of Formula I with a solution of an alkali or alkaline earth metal salt having a sufficiently basic anion (e.g. hydroxide, alkoxide, carbonate or hydroxide). Quaternary amine salts can be made by similar techniques.

Salts of compounds of Formula I can also be prepared by exchange of one cation to another. Cationic exchange can be effected by direct treatment of an aqueous solution of a salt of a compound of Formula I, (e.g, alkali metal or quaternary amine salt) with a solution containing the cation to be exchanged. This method is most effective when the desired salt containing the exchanged cation is insoluble in water, e.g., a copper salt, and can be separated by filtration.

Exchange may also be effected by passing an aqueous solution of a salt of a compound of Formula I, (e.g., an alkali metal or quaternary amine salt) through a column packed with a cation exchange resin containing the cation to be exchanged. In this method, the cation of the resin is exchanged for that of the original salt and the desired product is eluted from the column. This method is particularly useful when the desired salt is water-soluble, e.g., a potassium, sodium or calcium salt.

Acid addition salts, useful in this invention, can be obtained by reacting a compound of Formula I, with a suitable acid, e.g., p-toluenesulfonic acid, trichloroacetic acid or the like.

The compounds of formula I are active as herbicides and therefore, in a further aspect the invention provides a process for severely damaging or killing unwanted plants which process comprises applying to the plants, or to the growth medium of the plants, an effective amount of a compound of formula I as hereinbefore defined.

The compounds of formula I may be applied directly to the plant (post-emergence application) or to the soil before the emergence of the plant (pre-emergence application).

The compounds of formula I may be used on their own to regulate the growth of plants but in general are preferably used in the form of a composition comprising a compound of the invention in admixture with a carrier comprising a solid or liquid diluent. Therefore, in a still further aspect the invention provides plant growth regulating compositions comprising a compound of formula I as hereinbefore defined and an inert carrier therefor.

Compositions according to the invention include both dilute compositions, which are ready for immediate use, and concentrated compositions, which require to be diluted before use, usually with water. Preferably the compositions contain from 1 ppm to 2% of active ingredient, while concentrated compositions may contain from 20 to 99% of active ingredient, although from 20 to 70% is usually preferred.

The solid compositions may be in the form of granules, or dusting powders wherein the active ingredient is mixed with a finely divided solid diluent such as kaolin, bentonite, kieselguhr, dolomite, calcium carbonate, talc, powdered magnesia, Fuller's earth or gypsum. They may also be in the form of dispersible powders or grains, comprising a wetting agent to facilitate the dispersion of the powder or grains in liquid. Solid compositions in the form of a powder may be applied as foliar dusts.

Liquid compositions may comprise a solution or dispersion or an active ingredient in water optionally containing a surface-active agent, or may comprise a solution or dispersion of an active ingredient in a water-immiscible organic solvent which is dispersed as droplets in water.

The rate of application of the compounds of the invention will depend on a number of factors including, for example, the compound chosen for use, the identity of the plants to be treated, the formulations selected for use and whether the compound is to be applied for foliage or root uptake. As a general guide, however, an application rate of from 0.001 to 10 kilograms, per hectare is suitable while from 0.01 to 5 kilograms per hectare may be preferred.

The compositions of the invention may comprise, in addition to one or more compounds of the invention, one or more compounds not of the invention but which possess biological activity. In certain applications it may be preferred to use a herbicidal composition comprising a mixture of at least one herbicidal compound of formula I as hereinbefore defined and at least one other herbicide.

The compounds of this invention and their preparation are further illustrated by the following examples.

Example 1

6-Chloro-3-methyl-5-nitro-4(3H)-quinazolinone

A suspension of 6-chloro-5-nitro-4(3H)-quinazolinone (9.5 g, 4.2 x $10^{-2}$ mole), methyl iodide (6.0 g) and potassium carbonate (6.2 g, 4.2 x $10^{-2}$ mole) in acetone (100 ml) was boiled for 3 h. The solvent was then evaporated, water (100 ml) was added and the suspension stirred at room temperature for 30 min. Filtration afforded the desired methyl quinazolinone (8.2 g, 82%) as a colourless solid. $\delta^1$H ($d_6$-DMSO) 8.46 (s, 1H); 8.06 (d, J 9Hz, 1H); 7.88 (d, J 9Hz, 1H); 3.59 (s, 3H).

Example 2

6-chloro-3-methyl-5-phenylmethylthio-4-(3H)-quinazolinone

Lithium hydroxide monohydrate (11.1 g, 1.7 x $10^{-1}$ mole) was added portionwise over 15 min to a stirred solution of the nitro quinazolinone from Example 1 and benzyl mercaptan (19.5 ml, 1.7 x $10^{-1}$ mole) in dry N,N-dimethylformamide (100 ml) under a nitrogen atmosphere all at ice bath temperature. The cooling bath was then removed and the reaction stirred at room temperature overnight. Water (500 ml) was added and following a further 30 min stirring at room temperature the resulting suspension was filtered, washed with water, then ethanol before drying to afford the benzylthio ether (38 g, 78%) as a colourless solid. $\delta$ $^1$H ($d_6$-DMSO) 8.41 (s, 1H); 7.82 (d, J 9Hz, 1H); 7.53 (d, J 9Hz, 1H); 7.17 (brs, 5H); 4.20 (s, 2H); 3.50 (s, 3H).

Example 3

6-chloro-3-methyl-4-oxo-(3H)quinazoline-5-sulphonylchloride

Chlorine gas was bubbled gently into a stirred suspension of the benzylthioether (7.5 g, 2.5 x $10^{-3}$ mole) from Example 2 in acetic acid/water (1.1 100 ml) over 30 mins, whilst maintaining the internal temperature below 5°C. Water (100 ml) was added and the resulting suspension was filtered, washed well with water and dried to afford the sulphonyl chloride (6.7 g, 93%) as a colourless solid. $\delta$ $^1$H ($d_6$-DMSO) 9.20 (s, 1H); 7.85 (d, J 9Hz, 1H); 7.65 (d, J 9Hz, 1H); 3.51 (s, 3H).

Example 4

6-chloro-3-methyl-4-oxo-(3H)quinazoline-5-sulphonamide

Dry ammonia gas was bubbled gently into a stirred suspension of the sulphonyl chloride (19.0 g, 6.5 x $10^{-2}$ mole) in dry acetonitrile (300 ml) cooled to °C for 1 hour. The solvent was then evaporated and water

(300 ml) added. The resulting suspension was filtered, washed with water and dried to yield the sulphonamide (16.0 g, 90%) as a colourless solid. $\delta^1H(d_6$-DMSO) 8.52(s, 1H); 7.95(d, J 9Hz, 1H); 7.80 (d, J 9Hz, 1H); 7.64 (brs, 2H), 3.53 (s, 3H).

Example 5

3-methyl-4-oxo-(3H)quinazoline-5-sulphonamide

A suspension of the chlorosulphonamide (12.0 g, 4.4 x $10^{-2}$ mole) prepared in example 4, 10% Pd-C (4.0 g), ammonium formate (3.0 g, 4.8 x $10^{-2}$ mole) and sodium acetate (4.0 g, 4.8 x $10^2$ mole) in N,N-dimethylformamide (400 ml) was shaken under a hydrogen atmosphere at 50 psi for 3 h. The reaction mixture was then filtered and the solvent evaporated to afford a viscous oil. Water (200 ml) was added and the resulting suspension then filtered to afford the dechlorinated sulphonamide (9.2 g, 88%) as a colourless solid. $\delta^1H$ ($d_6$ - DMSO) 8.54(s, 1H); 8.30-8.10 (m, 1H);17.98-7.93 (m, 2H); 7.44 (brs, 2 H); 3.56 (s, 3H).

Example 6

N-[(4,6-Dimethoxypyrimidin-2-yl)amino carbonyl]-3-methyl-4-oxo-(3H)quinazoline-5-sulfonamide

To a stirred suspension of the sulphonamide prepared in example 5 (1.5 g, 6.3 x $10^{-3}$ mole) and O-phenyl-N-(4,6-dimethoxypyrimidin-2-yl) carbamate (1.8 g, 6.9 x $10^{-3}$ mole) in dry acetonitrile (75 ml) at 0°C was added 1,8-diazabicyclo[5.4.0]undec-7-ene (1 ml, 6.9 x $10^{-3}$ mole). The cooling bath was removed and the resulting solution stirred at room temperature overnight. The solvent was then evaporated affording a viscous oil which was dissolved in water (50 ml). The stirred solution was then acidified (pH 4-5) with aqueous citric acid (60 g/l). The suspension obtained was filtered, washed with water, then ether and dried to afford a colourless solid of sulphonyl urea (2.6 g, quantitative). $\delta^1H$ ($d_6$-DMSO) 12.58 (brs, 1H); 10.42 (brs, 1H); 8.47 (s, 1H); 8.44 - 8.33 (m, 1H); 8.05-8.00 (m, 2H); 5.96 (s, 1H); 4.01 (s, 6H); 3.76 (s, 3H).

Example 7

6-Diethylamino-3-methyl-4-oxo-(3H)quinazoline-5-sulfonamide

A mixture of 6-chloro-3-methyl-4-oxo-(3H)quinazoline-5-sulfonamide (0.5 g, 1.8 mmol) and diethylamine (0.4 ml, 3.8 mmol) in dimethylformamide (5 ml) was heated at 60°C for 1.5 hr. The solvent was removed by distillation and water was added to the residue. The resulting suspension was filtered and the solid was rinsed with water, dried in air to give the product (0.2 g, 36%) as a colourless solid.

Example 8

6-Phenylthio-3-methyl-4-oxo-(3H) quinazoline-5-sulfonamide was prepared from 6-chloro-3-methyl-4-oxo-(3H) quinazoline-5-sulfonamide following a similar procedure to that described in Example 7.

Example 9

Compounds Nos. 18, 19, 22, 23, 24, 63, 85 and 86 from Table 1 and Nos. 6 and 7 from Table 4 were each prepared by reaction of the O-phenyl carbamate of the appropriate 2-aminopyrimidine with the requisite sulfonamido-quinazoline according to the conditions described in Example 6.

The compounds were characterised by their proton magnetic resonance spectra which are recorded in Example 10.

Example 10

| Compound No. (Table No. 1) | Proton Magnetic Resonance Spectrum Chemical Shift δ in ppm |
|---|---|
| 22 | ($d_6$-DMSO) 9.62 (brs, 1H); 8.37 (s, 1H); 7.96 (d,J 8Hz, 1H); 7.72 (d,J 8Hz, 1H); 6.39 (s, 1H); 3.36 (s, 3H); 3.33 (s, 3H); 2.35 (s, 3H). |
| 23 | ($d_6$-DMSO) 12.71 (brs, 1H); 10.53 (brs, 1H); 8.48 (s, 1H); 8.02 (d,J8 Hz, 1H); 7.87 (d,J 8Hz, 1H); 6.00 (s, 1H); 3.99 (s, 3H); 3.33 (s, 3H). |
| 19 | ($d_6$-DMSO) 12.58 (brs, 1H); 10.42 (brs, 1H); 8.47 (s, 1H); 8.44-8.33 (m, 1H); 8.05-8.00 (m, 2H); 5.96 (s, 1H); 4.01 (s, 6H); 3.76 (s, 3H). |
| 18 | ($d_6$-DMSO) 13.18 (brs, 1H); 10.39 (brs, 1H); 8.47 (s, 1H); 8.43-8.33 (m, 1H); 8.03-7.98 (m, 2H(; 6.53 (s, 1H); 4.00 (s, 3H); 3.42 (s, 3H); 3.35 (s, 3H). |
| 24 | ($d_6$-DMSO) 7.86 (d,J 8Hz 1H); 7.37 (s, 1H); 7.29 (d,J 8Hz, 1H); 3.81 (s, 3H); 3.346 (s, 3H); 2.21 (s, 3H). |

| | |
|---|---|
| 63 | ($d_6$-DMSO) 12.78 (brs, 1H); 10.48 (brs, 1H); 8.52 (s, 1H); 8.00-7.93 (m, 2H); 5.98 (s, 1H); 5.23 (s, 1H); 3.97 (s, 6H); 3.17 (s, 3H). |
| 21 | ($d_6$-DMSO) 11.10 (brs, 1H); 9.77 (brs, 1H); 8.38 (s, 1H); 7.86 (d, J 8Hz, 1H); 7.43 (d, J 8Hz, 1H); 6.65 (s, 1H); 3.70 (s, 3H); 2.23 (s, 6H). |
| 85 | ($d_6$-DMSO) 11.10 (brs, 1H); 9.83 (brs, 1H); 8.38 (s, 1h); 7.60 (s, 2H); 5.91 (s, 1H); 3.91 (s, 3H); 3.11-3.05 (m, 4H); 1.28-1.13 (m, 6H). |

Example 11

This non-limiting Example illustrates the preparation of formulations of the compounds of the invention.

a) Emulsifiable Concentrate

Compound No 18 (Table 1) was dissolved in toluene/DMSO containing 7% v/v "Teric" N13 and 3% v/v "Kemmat" SC15B to give an emulsifiable concentrate which was diluted with water to the required concentration to give an aqueous emulsion which was applied by spraying.
("Teric" is a Trade Mark and "Teric" N13, is a product of ethoxylation of nonylphenol; "Kemmat" is a Trade Mark and "Kemmat" SC15B is a formulation of calcium dodecylbenzenesulfonate.)

b) Aqueous Suspension

Compound No 18 from Table 1 (5 parts by weight) and "Dyapol" PT (1 part by weight) were added to a 2% aqueous solution (94 parts by weight) of "Teric" N8 and the mixture was ball milled to produce a stable aqueous suspension which may be diluted with water to the required concentration to give an aqueous suspension which may be applied by spraying.
("Dyapol" is a Trade Mark and "Dyapol" PT is an anionic suspending agent; "Teric" N8 is a product of ethoxylation of nonylphenol.)

c) Emulsifiable Concentrate

Compound No 18 from Table 1 (10 parts by weight), "Teric" N13 (5 parts by weight) and "Kemmat" SC15B (5 parts by weight) were dissolved in "Solvesso" 150 (80 parts by weight) to give an emulsifiable concentrate which may be diluted with water to the required concentration to give an aqueous emulsion which may be applied by spraying.

("Solvesso" is a Trade Mark and "Solvesso" 150 is a high boiling point aromatic petroleum fraction).

d) Dispersible Powder

Compound No. 18 from Table 1 (10 parts by weight), "Matexil" DA/AC (3 parts by weight), "Aerosol" OT/B (1 part by weight) and china clay 298 (86 parts by weight) were blended and then milled to give a powder composition having a particle size below 50 microns.
("Matexil" is a Trade Mark and "Matexil" DA/AC is the disodium salt of a naphthalenesulfonic acid/formaldehyde condensate; "Aerosol" is a Trade Mark and "Aerosol" OT/B is a formulation of the dioctyl ester of sodium sulfosuccinic acid.)

e) Dusting Powder

Compound No 18 from Table 1 (10 parts by weight), attapulgite (10 parts by weight) and pyrophyllite (80 parts by weight) were throughly belended and then ground in a hammer-mill to produce a powder of particle size less than 200 microns.

Emulsifiable concentrates and/or suspensions of the compounds of the invention were prepared essentially as described in part a), b) or c) above and then diluted with water, optionally containing surface active agent and/or oil, to give aqueous compositions of the required concentration which were used, as described in Examples 12 and 13, in the evaluation of the pre-emergence and post-emergence herbicidal activity of the compounds.

Example 12

The pre-emergent herbicidal activity of the compounds of the invention formulated as described in Example 11 was assessed by the following procedure:

The seeds of the test species were sown in rows 2 cm deep in soil contained in seed boxes. The monocotyledonous plants and the dicotyledonous plants were sown in separate boxes and after sowing the two boxes were sprayed with the required quantity of a composition of the invention. Two duplicate seed boxes were prepared in the same manner but were not sprayed with a composition of the invention and were used for comparison purposes. All the boxes were placed in a glass house, lightly watered with an overhead spray to initiate germination and then sub-irrigated as required for optimum plant growth. After three weeks the boxes were removed from the glass house and the effect of the treatment was visually assessed. The results are presented in Table 7 where the damage to plants is rated on a scale of from 0 to 5 where 0 represents from 0 to 10% damage, 1 represents from 11 to 30% damage, 2 represents from 31 to 60% damage, 3 represents from 61 to 80% damage, 4 represents from 81 to 99% damage and 5 represents 100% kill. A dash (-) means that no experience was carried out.

The names of the test plants are as follows:

| | |
|---|---|
| Wh | Wheat |
| Ot | Wild Oats |
| Rg | Ryegrass |
| Jm | Japanese millet |
| B | Barley |
| P | Peas |
| Ip | Ipomea |
| Ms | Mustard |
| Sf | Sunflower |

TABLE 7

| Pre-emergent Herbicidal Activity | | | | | | | | | | |
|---|---|---|---|---|---|---|---|---|---|---|
| Compound No. Table 1 | Application Rate Kg/ha | Wh | Jm | Rg | O+ | B | P | Ip | Ms | Sf |
| 18 | 0.4 | 0 | 0 | 0 | 0 | 0 | 0 | 0 | 0 | 0 |
| 19 | 0.4 | 4 | 0 | 0 | 1 | 1 | 0 | 2 | 4 | 2 |
| 22 | 0.4 | 0 | 0 | 0 | 0 | 0 | 0 | 5 | 3 | 5 |
| 23 | 0.4 | 0 | 0 | 0 | 0 | 0 | 0 | 0 | 4 | 4 |
| 24 | 0.4 | - | 3 | 3 | 4 | 0 | 0 | 0 | 0 | 0 |
| 63 | 0.4 | 3 | 0 | 0 | 0 | 4 | 3 | 2 | 4 | 4 |

Example 13

The post-emergent herbicidal activity of the compounds of the invention formulated as described in Example 11 was assessed by the following procedure.

The seeds of the test species were sown in rows 2 cm deep in soil contained in seed boxes. The monocotyledonous plants ad the dicotyledonous plants were sown in separate seed boxes in duplicate. The four seed boxes were placed in a glass house, lightly watered with an overhead spray to initiate germination and then sub-irrigated as required for optimum plant growth. After the plants had grown to a height of about 10 to 12.5 cm one box of each of the monocotyledonous plants and the dicotyledonous plants was removed from the glass house and sprayed with the required quantity of a composition of the invention. After spraying the boxes were returned to the glass house for a further 3 weeks and the effect of treatment was visually assessed by comparison with the untreated controls. The results are presented in Table 8 where the damage to plants is rated on a scale of from 0 to 5 where 0 represents from 0 to 10% damage, 1 represents from 11 to 30% damage, 2 represents from 31 to 60% damage, 3 represents from 61 to 80% damage, 4 represents from 81 to 99% damage and 5 represents 100% kill. A dash (-) means that no experiment was carried out.

The names of the test plants are as follows:

| | |
|---|---|
| Wh | Wheat |
| Ot | Wild Oats |
| Rg | Ryegrass |
| Jm | Japanses millet |
| B | Barley |
| P | Peas |
| Ip | Ipomea |
| Ms | Mustard |
| Sf | Sunflower |

TABLE 8

| Pre-emergent Herbicidal Activity | | | | | | | | | | | |
|---|---|---|---|---|---|---|---|---|---|---|---|
| Compound No. Table 1 | Application Rate Kg/ha | Wh | Jm | Rg | Ot | B | P | Ip | Ms | Sf | Mz |
| 18 | 0.4 | 0 | 0 | 0 | 0 | 0 | 0 | 2 | 0 | 2 | 1 |
| 19 | 0.4 | 4 | 5 | 0 | 5 | 4 | 3 | 3 | 3 | 3 | 0 |
| 22 | 0.4 | 0 | 0 | 0 | 0 | 0 | 0 | 1 | 0 | 0 | 0 |
| 23 | 0.4 | 2 | 2 | 0 | 3 | 4 | 0 | 0 | 4 | 4 | 0 |
| 24 | 0.4 | 0 | 0 | 0 | 0 | 0 | 0 | 0 | 0 | 0 | 0 |
| 63 | 0.4 | 1 | 3 | 0 | 1 | 3 | 3 | 2 | 4 | 4 | 4 |

## Claims

1. Compounds of formula I and salts thereof

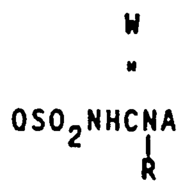

I

wherein Q is one of $Q_1$ to $Q_6$;

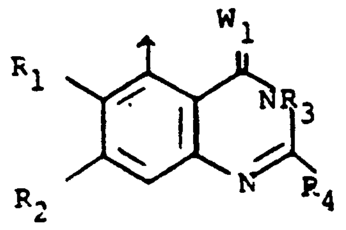

$Q_1$

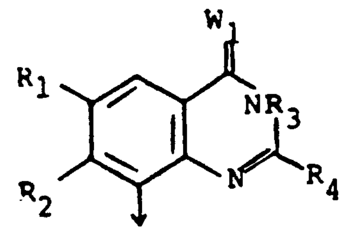

$Q_2$

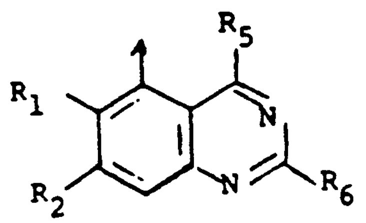

$Q_3$

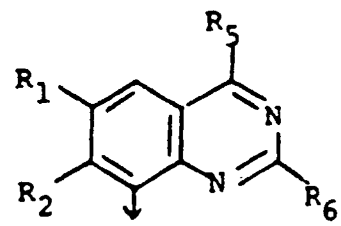

$Q_4$

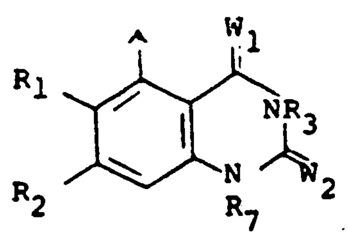

$Q_5$

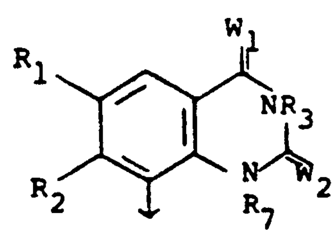

$Q_6$

wherein W, $W_1$ and $W_2$ are independently O or S: $R_1$ and $R_2$ are independently hydrogen, halogen, $C_1$-$C_4$ alkyl, $C_1$-$C_4$ haloalkyl, arylthio, aryloxy, nitro, $C_1$-$C_4$ alkoxy, $C_1$-$C_4$ haloalkoxy, cyano, carboxy, carbamoyl, N-($C_1$-$C_4$ alkyl)carbamoyl, N,N-di($C_1$-$C_4$ alkylthio, $C_1$-$C_4$ alkylsulfinyl, $C_1$-$C_4$ alkylsulfonyl, sulfamoyl, $C_1$-$C_4$ alkylsulfamoyl, di($C_1$-$C_4$ alkyl) sulfamoyl, amino, $C_1$-$C_4$ alkylamino or di($C_1$-$C_4$ alkyl) amino, $C_1$-$C_4$ acylamino;

R and $R_7$ are each independently selected from hydrogen, optionally substituted $C_1$-$C_4$ alkyl, $C_2$-$C_4$ alkenyl, $C_2$-$C_4$ alkynyl, or optionally substituted aryl or arylalkyl;

$R_3$ is selected from hydrogen, optionally substituted $C_1$-$C_4$ alkyl, $C_2$-$C_4$ alkenyl, $C_2$-$C_4$ alkynyl, optionally substituted aryl or arylalkyl, or $C_2$-$C_5$ alkoxyalkyl;

$R_4$, $R_5$ and $R_6$ are each independently selected from hydrogen, halogen, $C_1$-$C_4$ alkoxy, haloalkoxy, aryloxy, optionally substituted $C_1$-$C_4$ alkyl, $C_2$-$C_4$ alkynyl or optionally substituted aryl or arylalkyl, amino, $C_1$-$C_4$ alkylamino or di($C_1$-$C_4$ alkyl)amino, arylamino, alkylthio, haloalkylthio and arylthio;

A is 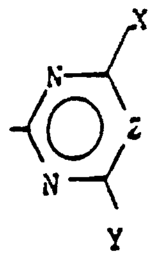, 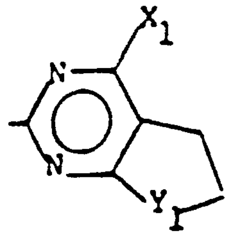, 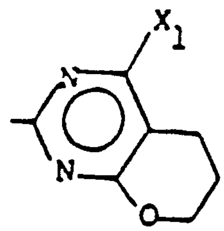, 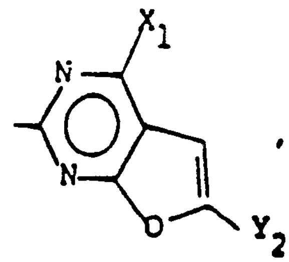,

A-1 A-2 A-3 A-4

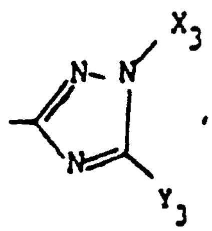, 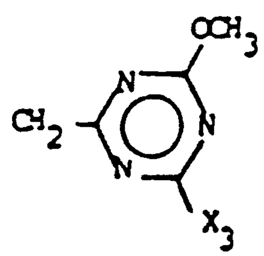 or 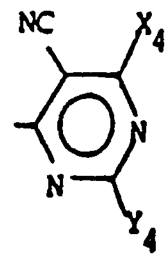

A-5 A-6 A-7

X is selected from $CH_3$, $OCH_3$, $OCH_2CH_3$, Cl, F, Br, I, $OCF_2H$, $CH_2F$ or $CF_3$; Y is selected from H, $CH_3$, $OCH_3$, $OC_2H_5$, $CH_2OCH_3$, $NH_2$, $NHCH_3$, $N(CH_3)_2$, $C_2H_5$, $CF_3$, $SCH_3$, $N(OCH_3)CH_3$, $OCH_2CH=CH_2$, $OCH_2C=CH$, $OCH_2CF_3$, cyclopropyl, $OCH_2CH_2OCH_3$, $CH_2SCH_3$, $(C=O)R_8$, $CR_8(QCH_3)_2$,

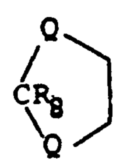 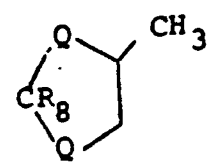

$CR_8(OCH_2CH_3)_2$, $OCF_2H$, $SCF_2H$, C = CH or C = $CCH_3$ where Q is selected from O or S and $R_8$ is H or $CH_3$; Z is selected from CH, N, $CCH_3$, $CC_2H_5$, CCl or CBr; $Y_1$ is O or $CH_2$; $X_1$ is $CH_3$, $OCH_3$, $OC_2H_5$ or $OCF_2H$; $Y_2$ is H or $CH_3$; $X_2$ is $CH_3$, $C_2H_5$ or $CH_2CF_3$; $Y_3$ is $OCH_3$, $OC_2H_5$, $SCH_3$, $SC_2H_5$, $CH_3$ or $CH_2CH_3$; $X_3$ is $CH_3$ or $OCH_3$; $Y_4$ is $CH_3$, $OCH_3$, $OCH_2CH_3$ or Cl; and $X_4$ is $CH_3$, $OCH_3$, $OCH_2CH_3$, $CH_2OCH_3$ or Cl.

2. A compound according to claim 1, wherein W is oxygen and A is A-1.

3. A compound according to claim 1, wherein R is hydrogen; $R_1$ and $R_2$ are independently hydrogen, fluorine, chlorine, bromine, methyl, methoxy, trifluoromethyl, methoxycarbonyl or methylthio; and Y is methyl, methoxy, ethoxy, methoxymethyl, trifluoromethyl, 2,2,2-trifluoroethoxy, dimethoxymethyl or difluoromethoxy.

4. A compound according to claim 2, wherein Q is Q1 and

a) in Q1;

(i) $R_1$ is selected from hydrogen, chlorine, bromine, methoxy carbonyl nitro, methoxy, methylthio, diethylamino and phenylthio;

(ii) $R_2$ is hydrogen; and

(iii) $R_3$ is selected from hydrogen, methyl ethyl and methoxymethyl and

b) in A-1;

(i) X and Y are independently selected from methyl and methoxy; and

(ii) Z is selected from nitrogen and CH.

5. A process of preparing a compound according to claim 1 by reacting a compound for formulz XI with a compound of formula X

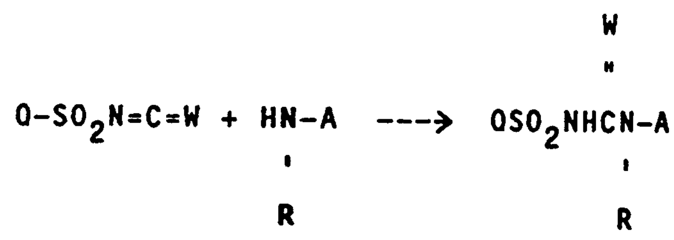

XI X I

where Q, W, R and A are as defined in claim 1.

6. A process of preparing a compound according to claim 1 wherein W is oxygen by reacting a compound of formula XII with a compound of formula X

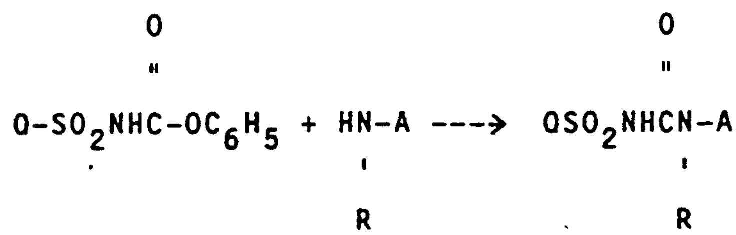

XII X Ia

where Q, R and A are as defined in claim 1.

7. A process of preparing a compound according to claim 1 wherein W is oxygen by reacting a compound of formula II with a compound of formula XIII

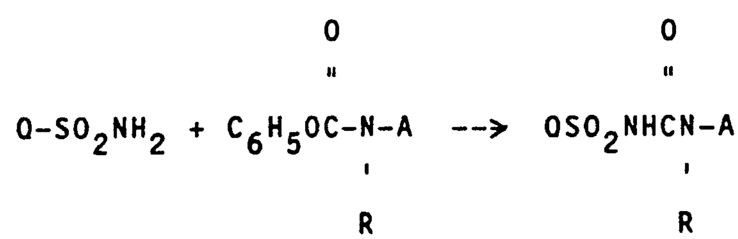

II XIII Ia

wherein Q, R and A are as defined in claim 1.

8. A process of preparing a compound according to claim 1, wherein R is hydrogen by reacting a compound of formula II with a compound of formula XIV

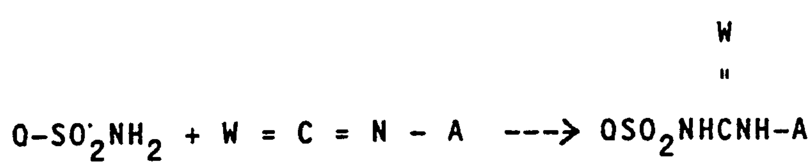

II XIV Ib

where Q, W and A are as defined in claim 1.

9. A herbicidal composition comprising a compound according to claim 1.

10. A process for severely damaging or killing unwanted plants, which process comprises applying to the plants or to the growth medium of the plants an effective amount of a compound according to claim 1.

European Patent Office

# EUROPEAN SEARCH REPORT

Application number

EP 90304124.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP - A1 - 0 128 116 (CIBA-GEIGY) * Claims 1,21,23,25 * -- | 1,9,10 | C 07 D 403/12<br>A 01 N 47/36 |
| A | EP - A2 - 0 108 708 (CIBA-GEIGY) * Claims 1,15-19 * -- | 1,9,10 | |
| A | EP - A1 - 0 107 979 (DU PONT) * Claims 1,30 * -- | 1,10 | |
| A | EP - A2 - 0 132 230 (CIBA-GEIGY) * Claim 20 * -- | 1 | |
| A | EP - A2 - 0 099 339 (CIBA-GEIGY) * Claims 1,29,30 * ---- | 1,9,10 | |

TECHNICAL FIELDS SEARCHED (Int Cl.5)

C 07 D 403/00

The present search report has been drawn up for all claims.

| Place of search | Date of completion of the search | Examiner |
|---|---|---|
| VIENNA | 21-06-1990 | HAMMER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document